# EUROPEAN PATENT SPECIFICATION

(11) **EP 1 258 731 B1**
(45) Date of publication and mention of the grant of the patent: **01.02.2006**
(21) Application number: 02008190.7
(22) Date of filing: 16.04.2002
(51) Int. Cl.: G01N 33/543, C12Q 1/68, B01J 19/00

(54) **Reactive solid support for DNA fragment detection**
Reaktiver Träger zur Bestimmung von DNA Fragmenten
Support solid réactif pour la détection de fragments d'ADN

(30) Priority: 20.04.2001 JP 2001122577; 05.06.2001 JP 2001168968; 05.06.2001 JP 2001168969; 05.06.2001 JP 2001168982; 05.06.2001 JP 2001168983
(43) Date of publication of application: 20.11.2002
(73) Proprietor: FUJI PHOTO FILM CO., LTD., Minamiashigara-shi, Kanagawa-ken 250-0193 (JP)
(72) Inventor: Inomata, Hiroko, Asaka-shi, Saitama 351-8585 (JP); Kojima, Masayoshi, Asaka-shi, Saitama 351-8585 (JP); Sudo, Yukio, Asaka-shi, Saitama 351-8585 (JP); Shinoki, Hiroshi, Asaka-shi, Saitama 351-8585 (JP); Seshimoto, Osamu, Asaka-shi, Saitama 351-8585 (JP)
(74) Representative: Albrecht, Thomas

(56) References cited:
- EP-A- 0 347 137
- EP-A- 1 179 363
- EP-A- 1 215 287
- WO-A-00/33078
- WO-A-00/61282
- WO-A-99/07751
- WO-A-99/09073
- US-A1- 2001 053 522

### SEQUENCE LISTING

<110> Fuji Photo Film Co., Ltd.
<120> REACTIVE SOLID SUPPORT AND DNA FRAGMENT DETECTION TOOL
<130> 12281EP
<140> EP 02 008 190.7
   <141> 2002-04-16
<150> 122577/2001
   <151> 2001-04-20
<150> 168968/2001
   <151> 2001-06-05
<150> 168969/2001
   <151> 2001-06-05
<150> 168982/2001
   <151> 2001-06-05
<150> 168983/2001
   <151> 2001-06-05
<160> 2
<170> PatentIn Ver. 2.1
<210> 1
   <211> 22
   <212> DNA
   <213> Artificial Sequence
<220>
   <223> Description of Artificial Sequence: sample oligonucleotide of which the 5' end is to be modified
<400> 1
   ctagtctgtg aagtgtctga tc 22
<210> 2
   <211> 40
   <212> DNA
   <213> Artificial Sequence
<220>
   <223> Description of Artificial Sequence: DNA fragment of which the 3' end and the 5' end are to be modified
<400> 2
   tcctccatgt ccggggagga tctgacactt caaggtctag 40

## Description

### Field of the Invention

The present invention relates to a detection tool useful for analyzing a structure of a biopolymer substance, and particularly relates to a detection tool useful for efficiently analyzing the expression, mutation, polymorphism and the like of a gene, wherein a large number of polymer substances originally from organisms or their analogs are aligned and fixed on the surface of a solid support having convex and concave portions. The present invention particularly relates to a high density array type detection tool (DNA detection chip) useful for analyzing a base sequence of a DNA fragment sample wherein a number of nucleotide derivatives or their analogs are aligned and fixed to the surface of a solid support having convex and concave portions, and a reactive solid support capable of advantageously being utilized for preparing the high density array type detection tool. Furthermore, the present invention relates to a chip useful for proteome analysis/proteomics wherein a protein or protein binding substance (except for nucleic acid) is fixed on the surface of the solid support, a method for detecting using said chip, and a method of manufacturing it.

### Background of the Invention

The technological development for efficiently analyzing gene functions of a variety of organisms has been rapidly progressed, and a detection tool called as a DNA chip where nucleotide derivatives such as a large number of DNA fragments or synthesized oligonucleotides or the like are fixed on the surface of a solid phase substrate has been employed in order to analyze the base sequence of the DNAs or DNA fragments. A molecule for detection such as a DNA or its fragments or a synthesized oligonucleotide like a nucleotide derivative bound to the surface of such a solid phase substrate is also referred to as a probe molecule. A representative DNA chip is a microarray in which a large number of probe molecules are aligned and fixed to a solid support such as a slide glass or the like. DNA chip related technologies relating to manufacturing of a DNA chip and its use are considered to be capable of also utilizable for detecting a biomolecules other than DNA. Therefore, these are expected to provide a new means for aiming at drug discovery research, development of a method of diagnosis of diseases or preventing the disease, or the like.

The DNA chip related technologies has been materialized since the fact that the method for determining the base sequence of a DNA by hybridization with an oligonucleotide was developed. Although this method could overcome the limitation of the method for determining the base sequence using gel electrophoresis, initially it did not come into practice.

Subsequently, by developing the DNA chip configured as described above and its preparation technique, the expression, mutation, polymorphism or the like of a gene has been capable of efficiently being examined in a short period of time. Specifically, a DNA fragment sample showing the complementarity to a DNA fragment or an oligonucleotide on the DNA chip prepared (which is also referred to as a target DNA fragment) is, in general, detected by utilizing the hybridization of the DNA fragment or the oligonucleotide on the DNA chip with the labeled DNA fragment sample.

In order to put the DNA chip preparation technique into practical use, a technology for aligning a large number of DNA fragments and oligonucleotides to surface of a solid support in high density and stable state is required.

As for a method for preparing a DNA chip, there are known a method for directly synthesizing oligonucleotide on the surface of the solid support (referred to as "on-chip method") and a method in which a DNA fragment or an oligonucleotide previously prepared is bound to the surface of the solid support. As for the on-chip method, a method for selectively synthesizing an oligonucleotide in the predetermined minute matrix region by combining use of a protective group selectively removable using photoirradiation, the photolithography technology used for fabricating a semiconductor device and a technology for synthesizing a solid phase (referred to as "masking technology") is representative.

As a method for binding and fixing a DNA fragment or an oligonucleotide previously prepared on the surface of the solid support, following methods are known corresponding to kinds of DNA fragments and kinds of solid supports.
(1) In the case where a DNA fragment to be fixed is a cDNA (complementary DNA synthesized by utilizing a mRNA as a template) or PCR products (DNA fragment obtained by amplifying the cDNA by a PCR method), in general, the cDNAs or PCR products are dotted to the surface of the solid support which was surface-treated with a polycationic compound (poly-lysine, polyethyleneimine or the like) using a spotter device provided in a DNA chip preparation device, and are electrostatically bound to the solid support by utilizing the charge held in the DNA fragments. As a method for treating the surface of the solid support, a method of employing a silane coupling agent containing an amino group, an aldehyde group, an epoxy group or the like is also utilized. In the surface treatment using the silane coupling agent, since the amino group, the aldehyde group or the like is fixed on the surface of the solid support by covalent bond, it is more stably fixed on the surface of the solid support, as compared with the case of surface treatment with a polycationic compound.
   As a modified method for utilizing the charge of the DNA fragments described above, there is reported a method wherein PCR products modified with an amino group is suspended in SSC (standard salt-citric acid buffer solution), dotted to the surface of the sililated slide glass, incubated, and then treated with sodium boron hydride and then with heating. However, there is a problem that it is difficult to necessarily obtain the sufficient fixation stability of the DNA fragments by this fixation method. In DNA chip technologies, detection limit is important. Therefore, binding and fixing of DNA fragments on the surface of the solid support in a sufficient amount (i.e. , in a high density) and in a stable state has a direct influence on increase of the detection limit of hybridization of DNA fragment probes and labeled nucleic acid fragments samples.
(2) In the case where an oligonucleotide (probe molecule) to be fixed is a synthesized oligonucleotide, there is known a method, in which an oligonucleotide into which a reactive group has been introduced is synthesized, then the oligonucleotide is dotted to the surface of the solid support surface-treated so as to previously form the reactive group, thereby binding and fixing the oligonucleotide to the surface of the solid support by covalent bond. For example, there are known a method in which an amino group-introduced oligonucleotide is reacted with the surface of the slide glass to which an amino group is introduced in the presence of PDC (p-phenylene diisothiocyanate), and a method in which an aldehyde group- introduced oligonucleotide is reacted with said slide glass. These two methods are more advantageous from the viewpoint that an oligonucleotide is stably bound and fixed to the surface of the solid support, as compared with the above-described method (1) for electrostatically binding by utilizing the charge of the DNA fragments. However, there are problems that in a method of performing the reaction in the presence of PDC, the reaction of PDC and an amino group-introduced oligonucleotide is slow, and in a method of using an aldehyde group-introduced oligonucleotide, the stability of Schiff base which is a reactive product is low (that is, hydrolysis is easily occurred).

In recent years, a technology employing an oligonucleotide analog which is referred to as PNA (peptide nucleic acid) instead of an oligonucleotide or a polynucleotide (also including a synthesized oligonucleotide or polynucleotide and a DNA molecule and DNA fragment, and a RNA molecule and RNA fragment) as a probe molecule of a DNA chip has also been proposed. As a method for fixing PNA to the solid phase substrate by covalent bond, a method of using the combination of avidin and biotin is also known (Japanese Unexamined Patent Publication No.H11-332595 gazette). In this publication gazette, a technology utilizing a surface plasmon resonance (SPR) biosenser as the solid phase substrate is also described. Utilizing the DNA chip in which a probe molecule is fixed on the surface plasmon resonance biosenser, a DNA fragment bound to its surface via hybridization can be detected by utilizing the surface plasmon resonance phenomenon.

Moreover, as a substrate of the DNA chip, use of a charge coupled device (CCD) is also known (Nucleic Acid Research, 1994, Vol. 22, No. 11, pp. 2124-2125).

In Japanese Unexamined Patent Publication No. H04-228076 gazette (corresponding to U. S. Patent No. 5,387,505), a technology for isolating a target DNA is describe. In this technology, the target DNA having biotin molecule is bound to a substrate, the surface of which an avidins molecule is fixed on.

Japanese Patent Publication No.H07-43380 gazette (corresponding to U. S. Patent No.5,094,962) describes a detection tool used for ligand-receptor assay, that is, an analyzing tool that an receptor molecule is bound to surface of a microporous polymer particle having a reactively active group on its surface.

On the other hand, in recent years, since the genomic analysis has been almost completed, the "proteome/proteomics" research, that provides essential information in order to finally understand meanings of the gene information and to simulate the life-activities of the cells, has been progressed. The term "proteome" means the all sets of proteins which are translated and produced in a specific cell, an apparatus and an organ, and the research field of high-level information analysis of chemical structure, total amount, expression period, modification after translation, formation of aggregation and the like are referred to as "proteomics".

The proteome research includes a profiling of proteins, an identification and precise analysis of proteins, a interaction network analysis and construction of a proteome data base, and is a field where these technologies are applied tc-the life science researches.

Among these, as the interaction network analysis method, a yeast two-hybrid method and a phage display method have been performed, and as a method of utilizing affinity capture, an immunoprecipitation method, a BIA-MA method, a column switching-mass spectrometry method and the like have been performed ("Proteome analysis method", pp. 163-211, published by Yodosha, Co., Ltd. ,2000). However, any of these interaction network analyses listed above has not achieved a high throughput analysis.

The report has been made by Schreiber et al. on a protein microarray for a high throughput analysis of interaction of proteins (Science 289: 1760-1763, 2000). This is a method in which protein aqueous solution is dotted to the slide glass having an aldehyde group, blocked with BSA solution, then reacted with the protein solution to carry out detection by a fluorescence scanner. In this case, there is a problem that the stability of Schiff base which is a reaction product between an aldehyde group and an amino group is low (usually, hydrolysis is easily occurred).

In addition to these, as a method of fixing protein to the solid phase, a method, in which a hydrophobic polypeptide is introduced into the end of the protein, is described in Japanese Patent Publication No.H07-53108 gazette.

In Japanese Patent No.2,922,040, a method of fixing an antibody protein with protein A molecule film is described.

In the international publication WO 00/61282,the description on a porous solid support is disclosed. The thickness from the supporting body is made in the range from 0.01 to 70 *µ*m and the porosity is in the range from 10 to 90% by coating particles mainly made of inorganic substances. When this porous solid support is employed, there is a merit that a fixation amount of an organism polymer is increased because its surface area is enlarged. However, in this case, there are problems such that (1) in the case where detection is carried out by fluorescence, luminescence, RI or the like, background level is increased since scattering by exciting light, emitting substances or the like is increased; (2) since impregnation amount of liquid at the time of spotting is increased, it is difficult to decrease the diameter of the spot, and to prepare a high density array; and (3) since the labeled specimen intrudes into a cavity between particles, it is necessary to sufficiently perform a washing step, and in the case of an insufficient washing, the background level is often increased.

European patent application 1 179 363, which belongs to the State of the art under Article 54(3) EPC, describes a micro-array for analysis of DNA is prepared by the steps of spotting onto a solid carrier in its predetermined area in which plural reactive groups are fixed an aqueous solution which contains a thickening agent and probe molecules (e.g., DNA fragments) having a group reactive with the reactive group of the solid carrier to produce covalent bonding; spotting onto the solid carrier in a different area having the same reactive groups an aqueous solution (same or different); incubating the aqueous solution-spotted solid carrier to produce the covalent bondings; and washing the solid carrier with an aqueous medium to remove the thickening agent from the solid carrier.

European patent application 1 215 287, which belongs to the State of the art under Article 54(3) EPC, describes a process for blocking a device for detection of biochemically active molecules is performed by the steps of:
bringing in the presence of an aqueous medium a detection device having probe molecules, ionic reactive groups, and non-ionic reactive groups on its surface, into contact with compounds which react with the non-ionic reactive groups to produce covalent bondings and compounds which form electrostatic bondings with the ionic reactive groups, simultaneously or separately;
   and
washing the surface of the detection device with an aqueous solvent or a water-miscible solvent.

European patent application 0 347 137 describes a water-insoluble microporous article comprises a microporous substrate having first and second outer surfaces. Affixed to at least one of those surfaces is a stabilized specific binding reagent admixed with certain hydrophilic, neutral or positively-charged binder materials. Particularly useful binder materials include certain quaternary polymers, vinyl-pyrrolidone polymers and acrylamide polymers. In this mixture, the reagent exhibits improved keeping stability compared to similar reagents used without binder materials. The reagent comprises water-in-soluble particles to which are attached receptor molecules to a target ligand. Substantially none of the reagent is entrapped within the microporous substrate. This article is useful for the detection of a target ligand in an assay involving the specific binding reaction of the ligand with correspondng receptor molecules, and can be included in a diagnostic test kit. It is particularly useful for the detection of Streptococcal antigen in a biological specimen when the receptor molecules are antibodies to that antigen.

International patent application WO 99/09073 relates to carrying out organic chemistry on solid supports comprising a functionalised amide or a functionalised sulphone, to functionalised supports comprising quaternary ammonium compounds, to a process for the preparation of tertiary amines or N-containing heterocyclic compounds, and to the use of said solid supports in the manufacture of combinatorial chemistry libraries or arrays of compounds.

International patent application WO 99/07751 describes a polymer having a side chain of general formula (I) in which R may be any suitable alkyl, oxyalkyl, aryl or oxyaryl linking group R is preferably C₁₋₆ alkyl (especially -CH₂-), a benzene group or a group -CH₂-O-Phe- Generally, the side chain will be attached to an ethylene moiety forming part of the backbone of the polymer. Preferred polymers include polystyrene. The polymer is useful as a support for solild phase chemical reactions especially combinatorial chemical synthesis.

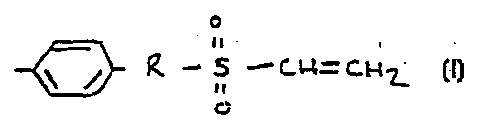

### Summary of the Invention

Objects of the present invention are to provide a reactive solid support capable of particularly advantageously being used for stably binding and fixing a previously prepared nucleotide derivative such as an oligonucleotide, a polynucleotide, or a peptide nucleic acid or their analogs in a high density state on the surface of the solid support, and to provide a detection tool for detecting DNA, RNA or their fragments having a specific base sequence portion, in which the nucleotide derivative or its analog has been bound and fixed on the reactive solid support.

Further objects of the present invention are to provide a chip in which at least one protein or protein binding substance is bound and fixed to a reactive solid support which can achieve rapid and stable binding and fixing, and to provide a detection method for detecting a specifically binding target substance by utilizing the chip.

The present invention provides the following (1) to (56).
(1) A reactive solid support having convex and concave portions on its surface, to which a group of vinyl sulfonyl groups or their reactive precursor groups are fixed by covalent bond via a linking group, respectively,
   wherein the convex and concave portions comprise particles formed by an inorganic substance, or the convex and concave portions are formed by an organic substance, wherein the convex and concave portions are generated by coating the solid support with a particulate substance.
(2) The reactive solid support of above (1), wherein the convex and concave portions comprise a particle of silicon, alumina or titanium having the average diameter of 50 *µ*m or less.
(3) The reactive solid support of above (2), wherein the organic substance is a macromolecular polymer or an aggregate of a macromolecular polymer.
(4) The reactive solid support of above (1), wherein a linked body of the vinylsulfonyl group or its reactive precursor group and the linking group is represented by the following formula:

   -L-SO₂-X

   in the above-described formula, X represents -CR¹=CR²R³ or -CHR¹-CR²R³Y, each of R¹, R² and R³ represents independently from each other an atom or a group selected from the group consisted of a hydrogen atom, an alkyl group having 1 to 6 carbon atoms, an aryl group having 6 to 20 carbon atoms, and an aralkyl group having 7 to 26 carbon atoms in total containing an alkyl chain having 1 to 6 carbon atoms; Y represents an atom or a group selected from the group consisted of a halogen atom, -OSO₂R¹¹, -OCOR¹², -OSO₃M and a quaternary pyridinium group; R¹¹ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; R¹² represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group.
(5) The reactive solid support of above (4), wherein X represents a vinyl group represented by -CH=CH₂.
(6) The reactive solid support of above (4), wherein L represents a linking group containing an atom of a bivalence or more except for carbon atom.
(7) The reactive solid support of above (4), wherein L represents a linking group having a linking portion selected from the group consisted of -NH-, -S- and -O-.
(8) The reactive solid support of above (4), wherein L represents a linking group represented by -(L¹)ₙ-NH-(CR¹R²)₂- or -(L¹)ₙ-S-(CR¹R²)₂- wherein R¹ and R² represents the same meanings as described above, L¹ represents a linking group, and n represents either 0 or 1.
(9) The reactive solid support of above (4), wherein L represents a linking group represented by -(L¹)ₙ-NHCH₂CH₂- wherein L¹ represents a linking group, and n represents either 0 or 1.
(10) The reactive solid support of above (8), wherein L¹ represents a linking group containing a group represented by -OSi- and n represents 1.
(11) The reactive solid support of above (1), wherein said solid support is a substrate in a sheet shape selected from the group consisted of a glass substrate, a resin substrate, a glass substrate or a resin substrate surface-treated with a silane coupling agent and a glass substrate or a resin substrate having a covering layer on its surface.
(12) The reactive solid support of above (11), wherein said solid support is a substrate in a sheet shape selected from the group consisted of a silicate glass substrate, a silicate glass substrate surface-treated with a silane coupling agent and a silicate glass substrate covered by an organic covering layer.
(13) A method of manufacturing the reactive solid support of above (4), wherein a disulfone compound represented by the following formula is brought into contact with a reactive solid support to the surface of which a reactive group is introduced:

   X¹-SO₂-L²-SO₂-X²

   in the above-described formula, each of X¹ and X² represents independently from each other -CR¹=CR²R³ or -CHR¹-CR²R³Y, each of R¹, R² and R³ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, -OSO₂R¹¹, -OCOR¹², -OSO₃M and a quaternary pyridinium group; R¹¹ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; R¹² represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L² represents a linking group.
(14) The method of manufacturing a reactive solid support of above (13), in which the reactive group introduced to the surface of said solid support is an amino group, a mercapto group or a hydroxyl group.
(15) A manufacturing method of a solid support comprising a nucleotide derivative or its analog bound to the surface of the support via a linking group having a sulfonyl group, wherein a surface of a reactive solid support having a convex and concave portion on which each of a group of vinylsulfonyl group or its reactive precursor group is fixed by covalent bond, is contacted with a nucleotide derivative or its analog having a reactive group which is capable of reacting with said reactive group.to form a covalent bond.
(16) The manufacturing method of above (15), wherein said nucleotide derivative or its analog is selected from the group consisted of an oligonucleotide, a polynucleotide and a peptide nucleic acid.
(17) The manufacturing method of above (15), wherein a reactive solid support that a linked body of a vinylsulfonyl group or its reactive precursor group represented by the following formula and a linking group is bound to and fixed on, is used as a reactive solid support having a convex and concave portion:

   -L-SO₂-X

   in the above-described formula, X represents -CR¹=CR²R³ or -CHR¹-CR²R³Y, each of R¹, R² and R³ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, -OSO₂R¹¹, -OCOR¹², -OSO₃M and a quaternary pyridinium group; R¹¹ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; R¹² represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group or a single bond.
(18) The manufacturing method of above (17), wherein X represents a reactive group represented by -CR¹=CR²R³ wherein each of R¹, R² and R³ represents the same meanings as described above.
(19) A solid support having a convex and concave portion, in which a nucleotide derivative or its analog obtained by a manufacturing method of any one of above (15) to (18) is bound and fixed to surface of the solid support.
(20) A method of binding and fixing a complementary oligonucleotide or polynucleotide, wherein the solid support having the convex and concave portion to which the nucleotide derivative or its analog is bound of above (19) is contacted with an oligonucleotide or a polynucleotide having complementarity to said fixed nucleotide derivative or its analog in the presence of an aqueous medium.
(21) The method of above (20), wherein a detectable label is bound to said complementary oligonucleotide or polynucleotide.
(22) A solid support to which a oligonucleotide or a polynucleotide having complementarity is bound and fixed wherein the solid support having the convex and concave portion to which to the nucleotide derivative or its analog is bound as in (19), a oligonucleotide or a polynucleotide having complementarity to said nucleotide derivative or its analog is bound in a complementary manner.
(23) The solid support of above (22), wherein a detectable label is bound to said oligonucleotide or polynucleotide having the complementarity.
(24) A method of identifying or screening a gene, wherein the solid support having a convex and concave portion to the surface of which the nucleotide derivative or its analog is bound and fixed as in above (18), or the solid support to which the complementary oligonucleotide or polynucleotide is bound and fixed as in above (20) is utilized.
(25) A biological material chip, wherein A, which represents a residue of at least one protein or protein binding substance, is bound to a solid support having a convex and concave portion by covalent bond via a sulfonyl group as the following formula (I):

   Solid support-L-SO₂-X-A (I)

   in the formula (I), L represents a linking group; X represents -CR¹(R²)-R³(R⁴)-; each of R¹, R², R³ and R⁴ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and A represents a residue of a protein or protein binding substance except for nucleic acid.
(26) The chip of above (25), wherein said protein or protein binding substance bounded to the surface is an antibody, an antibody fragment, a ligand, an antigen, a hapten or a receptor.
(27) The chip of above (25), wherein said protein or protein binding substance bound to the surface is avidins.
(28) The chip of above (27), in which avidins are an avidin, a streptoavidin or altered bodies thereof which are capable of forming a stable complex with a biotin.
(29) The chip of above (25), wherein said protein bound to the surface is a nucleic acid recognition protein.
(30) The chip of above (29), in which said nucleic acid recognition protein is a double stranded DNA recognition protein.
(31) The chip of above (30), wherein said double stranded DNA recognition protein is a double stranded DNA recognition antibody.
(32) The chip of above (30), wherein said double stranded DNA recognition protein is a DNA transcription factor.
(33) The chip of above (30), wherein said double stranded DNA recognition protein is a protein having a Zinc finger motif or a Ring finger motif.
(34) The chip of any one of above (25) to (33), wherein said convex and concave portion comprises a particle formed by an inorganic substance.
(35) The chip of above 36, wherein said convex and concave portion comprises a particle containing silicon, alumina or titanium of which average particle diameter is 50 µm or less.
(36) The chip of any of above (25) to (33), in which said convex and concave portion is formed by an organic substance.
(37) The chip of above (36), wherein said organic substance comprises a high molecular polymer or an aggregate of a high molecular polymer.
(38) The chip of above (25), wherein said solid support is a glass, a plastic, an electrode surface or a sensor chip surface.
(39) A method of detecting a target substance, comprising the steps of:
   contacting the chip of above (25) with a sample containing a target substance which specifically binds to said protein or protein binding substance except for nucleic acid supported on the surface of said chip; and
   detecting formation of reciprocal binding between said protein or protein binding substance and said target substance.
(40) The method of detecting a target substance of above (41), wherein said target substance is labeled with at least one component capable of generating a detectable signal.
(41) The method of detecting a target substance of above (41), comprising a step of performing blocking processing of the chip with an aqueous solution of an amino acid, a peptide or a protein.
(42) The method of manufacturing the chip of above (25) comprising a step in which a solid support having a convex and concave portion containing a vinylsulfonyl group or its reactive precursor group represented by the following formula (II) on its surface is contacted with at least one protein or protein binding substance having a reactive group which forms a covalent bond by reacting with said vinylsulfonyl group or its reactive precursor group:

   -L-SO₂-X' (II)

   in the above-described formula (II), L represents a linking group which binds -L-SO₂-X' to a solid support; X' represents -CR¹=CR²(R³) or -CH (R¹) -CR² (R³) (Y) ; each of R¹, R² and R³ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and Y represents a group which is substituted by a neucleophilic reagent or a group which is eliminated as a "HY" by a base
(43) The method of manufacturing a chip of above (42), wherein said protein or protein binding substance bound to the surface is an antibody, an antibody fragment, a ligand, an antigen, a hapten or a receptor.
(44) The method of manufacturing.a chip of above (42), wherein said protein or protein binding substance bound to the surface is avidins.
(45) The method of manufacturing a chip of above (44), wherein avidins are an avidin, a streptoavidin or altered bodies thereof which are capable of forming a stable complex with a biotin.
(46) The method of manufacturing a chip of above (42), wherein said protein bound to the surface is a nucleic acid recognition protein.
(47) The method of manufacturing a chip of above (46), wherein said nucleic acid recognition protein is a double stranded DNA recognition protein.
(48) The method of manufacturing a chip of above (47), wherein said double stranded DNA recognition protein is a double stranded DNA recognition antibody,
(49) The method of manufacturing a chip of above (47), wherein said double stranded DNA recognition protein is a DNA transcription factor.
(50) The method of manufacturing a chip of above (47), wherein said double stranded DNA recognition protein is a protein having a Zinc finger motif or a Ring finger motif
(51) The method of manufacturing a chip of above (42), wherein said convex and concave portion comprises a particle formed by an inorganic substance.
(52) The method of manufacturing a chip of above (34), wherein said convex and concave portion comprises a particle containing silicon, alumina or titanium of which average particle diameter is 50 *µ*m or less.
(53) The method of manufacturing a chip of above (42), in which said convex and concave portion is formed by an organic substance.
(54) The method of manufacturing a chip of above (53), wherein said organic substance is a high molecular polymer or an aggregate of a high molecular polymer.
(55) The method of manufacturing a chip of above (42), in which a solid support is a glass, a plastic, an electrode surface or a sensor chip surface.
(56) The method of manufacturing a chip of above (42), comprising the steps of:
   fixing at least one protein or protein binding substance to a solid support having convex and concave portions by contacting said protein or protein binding substance to said solid support; and
   performing blocking process of a free vinylsulfonyl group or its reactive precursor group located on the surface of said solid support with an amino acid, a peptide or a protein aqueous solution.

### Brief Description of the Drawings

The accompanying drawings, which are incorporated in and constitute a part of the specification, illustrate presently preferred embodiments of the invention, and together with the general description given above and the detailed description of the preferred embodiments given below, serve to explain the principles of the invention.
FIG. 1 is a schematic diagram showing a representative oligonucleotide fixed solid support of the present invention and a representative method of the present invention for fixing an oligonucleotide;
FIG. 2 is a schematic diagram showing the configuration of a protein chip, which is a representative embodiment of the present invention;

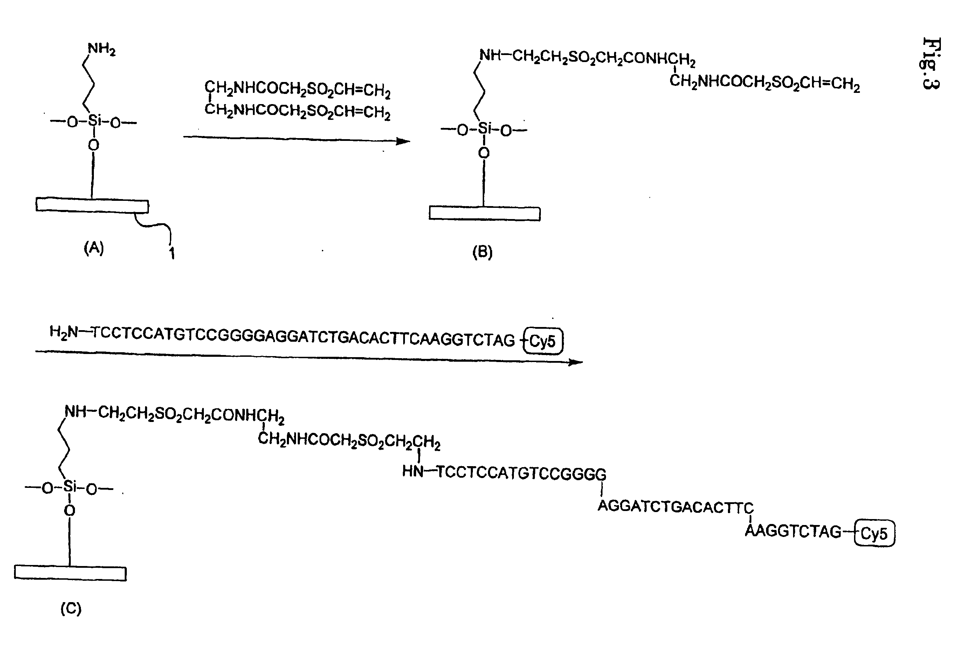

FIG. 3 is a schematic diagram showing a fixation method of Example 1; and
FIG. 4 is a graphical representation showing the results of detecting a ligand using an antibody fixed slide according to the present invention.

### Detailed Description of the Invention

A reactive solid support of the present invention has the configuration in which a group of vinyl sulfonyl groups or their reactive precursor groups are bound and fixed on the surface of the solid support having convex and concave portions via a linking group by covalent bond. The reactive solid support of the present invention can be manufactured by preparing the solid support having convex and concave portions to the surface of which a reactive group has been previously introduced, and bringing this solid support into contact with a compound having a reactive group capable of forming a covalent bond by reacting with a reactive group provided to the surface of the support at one of end portions or nearby the end portion and having a vinyl sufonyl group or a reactive precursor group of the vinyl sufonyl group at the other end portion or nearby the end portion.

It is preferable that the solid support is a particularly hydrophobic or lower hydrophilic substrate having a flat and smooth surface. Moreover, a substrate having a surface of lower degree of flatness with convex and concave portions can be also employed. As a substance for the solid support, a variety of porous substances such as glass, cement, ceramics or new ceramics such as potteries or the like, polymers such as polyethylene terephthalate, cellulose acetate, polycarbonate of bisphenol A, polystyrene, polymethyl methacrylate or the like, silicon, activated carbon, porous glass, porous ceramics, porous silicon, porous activated carbon, woven fabric, knitted fabric, non-woven fabric, filter paper, short fiber, membrane filter or the like can be listed. It is preferable that the size of a fine hole of a porous substance is in the range from 2 to 1000 nm, and particularly preferable in the range from 2 to 500 nm. It is particularly preferable that the substance of a solid support is a glass or silicon. This is because of the easiness of surface treatment and the easiness of analysis by an electrochemical method. It is preferable that the thickness of the solid support is in the range from 100 to 2000 *µ*m.

As for the solid support having convex and concave portions used for manufacturing a reactive solid support of the present invention, a variety of solid supports conventionally used for manufacturing DNA chips or proposed for manufacturing the DNA chip can be preferably utilized. As an example of such a solid support, a glass substrate, a resin substrate, a glass substrate or resin substrate surface treated by a silane coupling agent, or a glass substrate or a resin substrate having a covering layer on the surface, or the like can be listed. As for the solid support, a silicate glass substrate, a silicate glass substrate surface treated by a silane coupling agent, or a silicate glass substrate covered by an organic covering layer is particularly preferable. Moreover, it may be an electrode substrate used as a substrate of a DNA chip used for an electrochemical analyzing method. Moreover, it may be a variety of functional substrates such as a substrate used for the above-described surface plasmon resonance (SPR) biosensor, a charge coupled device (CCD) or the like. Furthermore, adding to these substances, a solid support in a particle shape can be also employed.

As a substance having convex and concave properties, a particle formed by an inorganic substance is preferable. For example, a metal oxide such as colloidal silica, alumina sol, titanium oxide, ZrO₂ or the like, a plastic having a hydroxyl group, latex or the like is listed. Preferably diameter of the particle is 50 *µ*m or less. Particularly in the case where a co-focal laser fluorescence detection is carried out, it is preferable to use particles whose diameter is 100 *µ*m or less in order to suppress the background level. As a method of coating a substance having the convex and concave properties on the solid support, a method can be used in which the substance having the convex and concave properties is mixed with a silane coupling agent, and brought into contact with the solid support to bring about reaction. Thus the convex and concave properties can be easily given to the solid support. Particularly in the case where the solid support is a glass, this method is particularly effective. It can be also prepared by bringing the substance having the convex and concave properties into contact with the solid support, drying it, and then treating it with a silane coupling agent.

Generation of the convex and concave portions by coating the solid support with a particulate substance provides considerably increased surface area compared with that of flat and smooth surface, resulting in an advantage such that bonded amount of DNA fragments to be fixed on the solid support can be increased to a large extent. Furthermore, the form of single layer is more advantageous than the form of laminated layers from the following reasons:
1. In the case where detection is carried our by fluorescence, luminescence, RI or the like, the scattering due to the exciting light, emitting substance or the like is decreased, and the background level is lowered.
2. Since the impregnation of the liquid at the time of spotting is slight, the diameter of spot is capable of being made smaller, and an array having a high density can be prepared.
3. In an operation after the hybridization, a post-treatment washing step for removing an un-reacted labeled specimen is easily performed. In the case of the laminated layers, the labeled specimen intrudes into the cavity between particles. Therefore, it is necessary to sufficiently carry out the washing step, and in the case where it is not sufficient, the background level may be often heightened.

As for an organic substance having the convex and concave properties, monosaccharides such as glucose, fructose and the like or their derivatives, polysaccharides such as dextran, amylose, starch and the like or their derivatives, for example, carboxymethyl starch, and high molecular polymers such as PVA, polyacrylic acid, cellulose, carboxymethyl cellulose, polyacetal and the like can be listed. Particularly, an organic substance having a hydroxyl group can form an aggregation by using it in combination with a silane coupling agent, and the convex and concave portions can be easily formed. The size of convex and concave portions (height) is preferably 50 *µ* m or less, particularly in the case where detection is carried out by the co-focus laser fluorescence, it is preferable to use the particle of 100 nm or less in order to suppress the background level.

Using an aminated silane coupling agent, it is possible to introduce both the convex and concave portions and an amino group to the surface at the same time. Further, using TMOS or TEOS, it is also possible to initially introduce the convex and concave portions, then to introduce the amino group to the surface by reacting the aminated silane coupling agent.

It is desirable to perform the covering treatment on the surface of the solid support having the convex and concave portions by a polymer containing an amino group such as a polycationic compound on side chain (for example, poly-L-lysine, polyethyleneimine, polyalkylamine or the like is preferable, and poly-L-lysine is more preferable) in order to bind and fix bifunctional reactive compounds such as divinyl sulfone compound or the like by covalent bond (in this case, the reactive group introduced to the surface of the solid support is an amino group). Alternatively, the surface of the solid support may be brought into contact and treated with a surface treatment agent having a reactive group such as a silane coupling agent which reacts with the surface of the solid support and a reactive group such as an amino group.

In the case where the covering treatment is performed with a polycationic compound, an amino group or a mercapto group is introduced to the surface of the solid support having the convex and concave portions by electrostatically binding between the polymer compound and the surface of the solid support. On the other hand, in the case where the surface treatment is performed by a silane coupling agent, the amino group or mercapto group stably exists on the surface of the solid support since it is bound and fixed to the surface of the solid support by covalent bond. In addition to an amino group or a mercapto group, an aldehyde group, an epoxy group, a carboxyl group or a hydroxyl group may be also preferably introduced.

As a silane coupling agent having an amino group, it is preferable to use γ-aminopropyltriethoxy silane, N-β (aminoethyl)-γ-aminopropyltrimethoxy silane, or N-β (aminoethyl)-γ-aminopropylmethyldimethoxy silane, and particularly it is preferable to use γ-aminopropyl triethoxy silane.

The treatment with a silane coupling agent may be performed in combination with the treatment using a polycationic compound. Using this method, an electrostatical interaction between a hydrophobic- or a low hydrophilic-solid support and DNA fragments can be promoted. A layer consisted of a hydrophilic polymer having a charge or the like or a layer consisted of a crosslinking agent may be further provided on the surface of the solid support treated by a polycationic compound. As a result of providing such a layer, the height of the convex and concave portions of the solid support treated by the polycationic compound can be reduced. Depending on the types of the solid supports, it is possible that a hydrophilic polymer is contained in the support, and the solid support subjected to such a treatment can be also preferably used.

On the surface of the usually utilized solid support for a DNA chip, a large number of regions previously fractioned or expected are set and provided, and in each region, as described above, a reactive group which is capable of reacting with bifunctional reactive compound such as divinyl sulfone compound or the like has been previously introduced. A reactive group such as the above-described amino group, mercapto group or hydroxyl group or the like is provided on the surface of each region of the solid support which is to be employed. However, on a solid support not having such a reactive group, as described above, the introduction of a reactive group is performed by surface treatment using a silane coupling agent, or by utilizing a method of coating and covering a polymer or the like having a reactive group such as an amino group on side chain on the surface of the solid support.

In the solid support equipped with a reactive group, as a result of bringing it into contact with a bifunctional reactive compound such as divinyl sulfone compound or the like, the reactive group and bifunctional reactive compound are reacted to form a covalent bond, the reactive group portion of the solid support is extended, and a reactive chain having a vinyl sulfonyl group or its reactive precursor group at its end or nearby the end is formed, thereby providing a reactive solid support of the present invention.

In a reactive solid support of the present invention, a linked body of a vinyl sulfonyl group or its reactive precursor group and a linking group introduced on the surface of the solid support is desirably a linked body represented by the following formula (1):

-L-SO₂-X (1)

In the above-described formula (1), X represents -CR¹=CR²R³ or -CHR¹-CR²R³Y (reactive precursor group). Each of R¹, R² and R³ represents independently from each other a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms. Examples of an alkyl group having 1 to 6 carbon atoms include a methyl group, an ethyl group, an n-propyl group, an n-butyl group, and an n-hexyl group. A methyl group is particularly preferable. Aryl groups include a phenyl group and a naphthyl group. It is preferable that each of R¹, R² and R³ represents a hydrogen atom, respectively.

Y represents a group which is substituted by a nucleophilic reagent such as -OH, -OR°, -SH, NH₃, NH₂R⁰ (wherein R⁰ represents a group such as alkyl group except for hydrogen atom), or a group which is eliminated as "HY" by base. Examples thereof include a halogen atom, -OSO₂R¹¹, -OCOR¹², -OSO₃M, or a quaternary pyridinium group (R¹¹ represents an alkyl group having 1 to 6 carbon atoms, an aryl group having 6 to 20 carbon atoms, or an aralkyl group having 7 to 26 carbon atoms in total containing an alkyl chain having 1 to 6 carbon atoms; R¹² represents an alkyl group having 1 to 6 carbon atoms or halogenated alkyl group having 1 to 6 carbon atoms; M represents a hydrogen atom, an alkaline metal atom, or an ammonium group) .

L represents a bivalent or more than bivalent linking group for linking the solid support or a linking group binding to the solid support with the above-described -SO2-X group. However, L may be a single bond. Examples of the bivalent linking groups include an alkylene group having 1 to 6 carbon atoms, an aliphatic cyclic group having 3 to 16 carbon atoms, an arylene group having 6 to 20 carbon atoms, a heterocyclic group having 2 to 20 carbon atoms containing 1 to 3 hetero atoms selected from the group consisted of N, S and P, a group containing one group or the combination of a plurality of groups selected from the group consisted of -O-, -S-, -SO-, -SO₂-, -SO₃-, -NR¹¹-, -CO- and their combinations are preferable. R¹¹ is preferably a hydrogen atom, an alkyl group having 1 to 15 carbon atoms, an aryl group having 1 to 6 carbon atoms, or an aralkyl group having 7 to 21 carbon atoms containing an alkyl group having 6 to 20 carbon atoms, and more preferably a hydrogen atom or an alkyl group having 1 to 6 carbon atoms, and particularly preferably a hydrogen atom, a methyl group or an ethyl group.

In the case where L represents a group containing the combination of two or more of the groups selected from the group consisted of -NR¹¹-, -SONR¹¹-, -CONR¹¹-, -NR¹¹COO-, and -NR¹¹CONR¹¹-, these R¹¹ may bind each other to form a ring.

An alkyl group of R11, an aryl group of R11 and an aralkyl group of R11 may have a substituent. Such substituents include an atom or a group selected from the group consisted of a hydroxyl group, an alkoxy group having 1 to 6 carbon atoms, an alkenyl group having 1 to 6 carbon atoms, a carbomoyl group having 2 to 7 carbon atoms, an alkyl group having 1 to 6 carbon atoms, an aralkyl group having 7 to 16 carbon atoms, an aryl group having 6 to 20 carbon atoms, an sulfamoyl group (or its Na salt, K salt or the like), a sulfo group (or its Na salt, K salt or the like), a carboxylic acid group (or its sodium salt, potassium salt or the like), a halogen atom, an alkenylene group having 1 to 6 carbon atoms, an arylene group having 6 to 20 carbon atoms, sulfonyl group and their combinations.

The preferable examples of the above-described "-X" group will be indicated below. Moreover, examples of a group which can be used as "-L-SO2-X" will be indicated as described later.

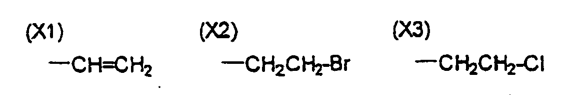

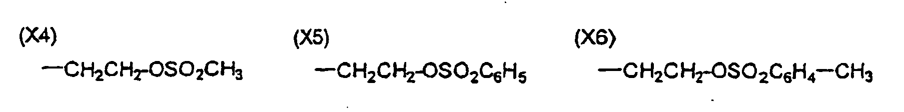

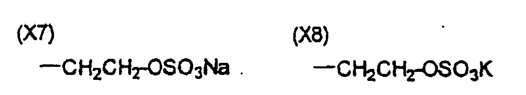

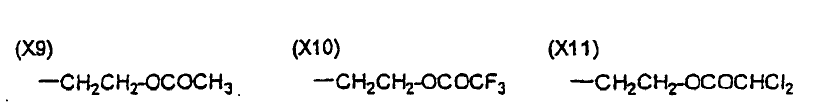

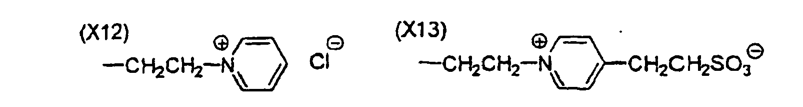

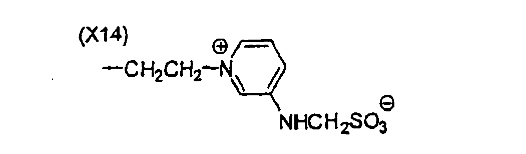

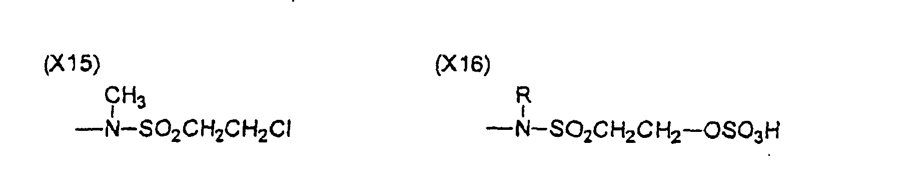

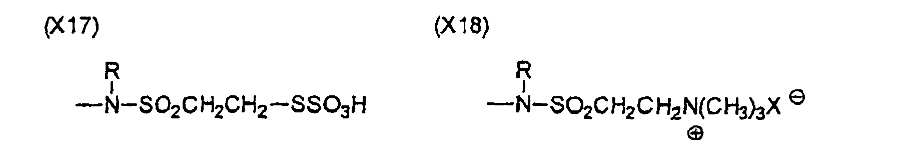

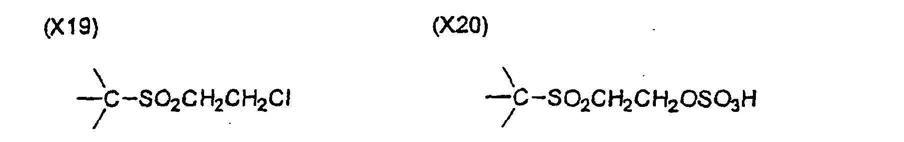

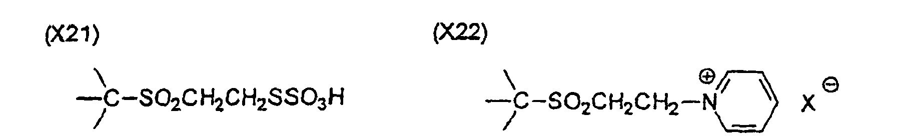

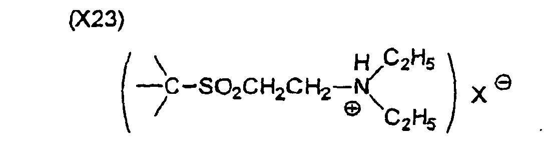

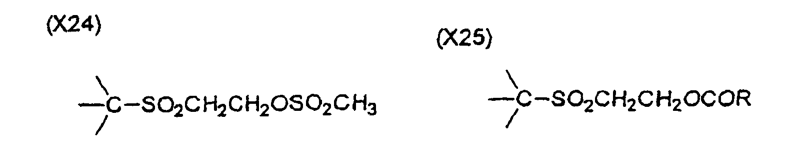

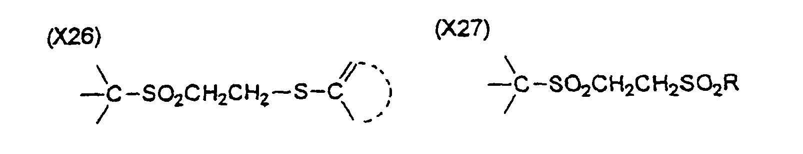

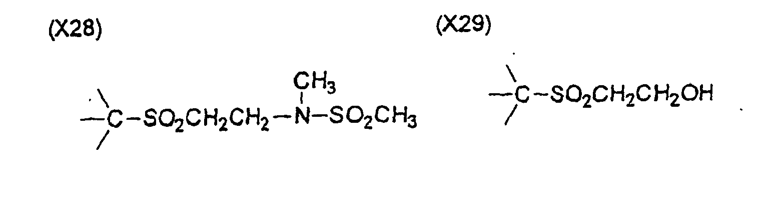

In the above-described examples, it is preferable that "-X" represents (X1), (X2), (X3), (X4), (X7), (X8), (X13) or (X14). It is more preferable that "-X" represents (X1) or (X2). It is particularly preferable that "-X" represents a vinyl group represented by (X1).

Preferable examples of L will be indicated below. "a" represents an integer of 1 to 6, preferably 1 or 2, and particularly preferably 1. "b" represents an integer of 0 to 6, and preferably either 2 or 3.

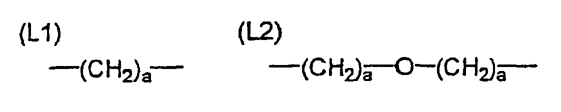

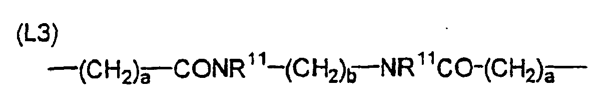

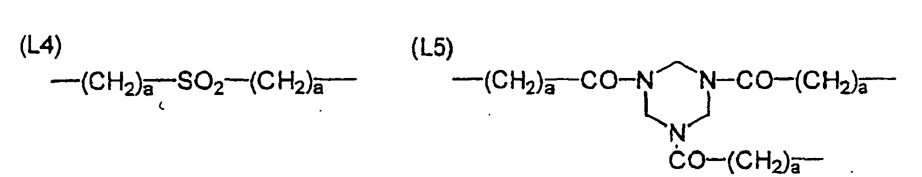

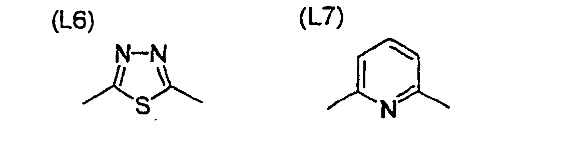

As for L, in addition to the above-described bivalent linking groups, a group that a hydrogen atom of the alkylene group in the above-described formula is substituted with a -SO₂CH=CH₂ group is also preferable.

As for a bifunctional reactive compound utilized for obtaining a solid support to which a vinyl sulfonyl group represented by the foregoing formula (1) or its reactive precursor group is fixed by covalent bond, a disulfone compound represented by the following formula (2) can be advantageously utilized.

X¹-SO₂-L²-SO₂-X² (2)

[In the above-described formula, each of X¹ and X² independently from each other represents -CR¹=CR²R³ or -CHR¹-CR²R³Y (reactive precursor group) ; each of R¹, R² and R³ independently from each other represent an atom or a group selected from the group consisted of a hydrogen atom, an alkyl group having 1 to 6 carbon atoms, an aryl group having 6 to 20 carbon atoms, and an aralkyl group having 7 to 26 carbon atoms in total containing an alkyl chain having 1 to 6 carbon atoms; Y represents an atom or a group selected from the group consisted of a halogen atom, -OSO₂R¹¹, -OCOR¹², -OSO₃M and quaternary pyridinium group; R¹¹ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; R¹² represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and ammonium group; and L² represents a linking group] .

Specifically, a reactive solid support of the present invention can be easily manufactured by bringing a disulfone compound represented by the above-described formula (2) into contact with the above mentioned solid support, for example in the aqueous atmosphere.

The representative examples of disulfone compound preferably used in the present invention will be shown in the followings. It should be noted that a disulfone compound might be used by mixing two types or more.

(S1) H₂C=CH―SO₂―CH₂―SO₂―CH=CH₂

(S2) H₂C=CH―SO₂―CH₂OCH₂―SO₂―CH=CH₂

(S3) H₂C=CH―SO₂―CH₂CH₂CH₂―SO₂―CH=CH₂

(S4) H₂C=CH―SO₂―CH₂CH(OH)CH₂―SO₂―CH=CH₂

(S5) H₂C=CH―SO₂―CH₂CONHCH₂CH₂NHCOCH₂―SO₂―CH=CH₂

(S6) H₂C=CH―SO₂―CH₂CONHCH₂CH₂CH₂NHCOCH₂―SO₂―CH=CH₂

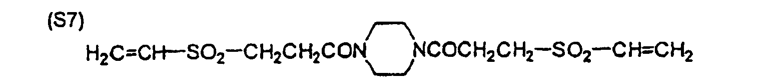

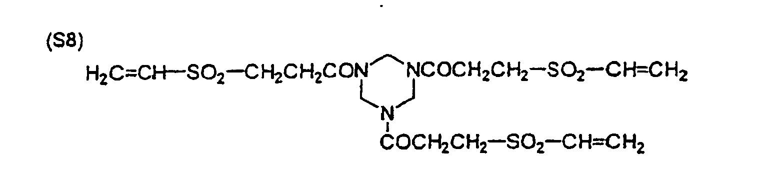

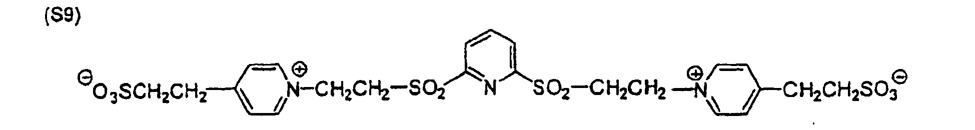

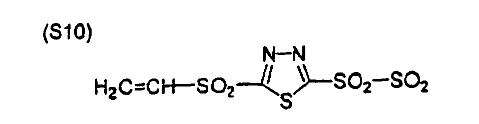

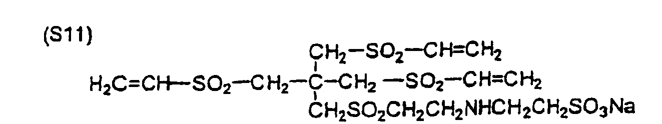

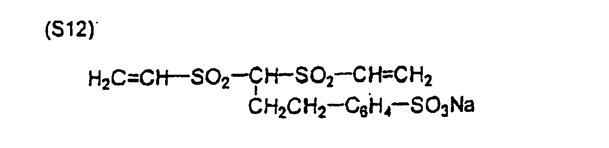

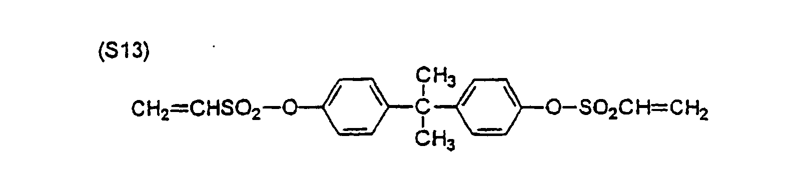

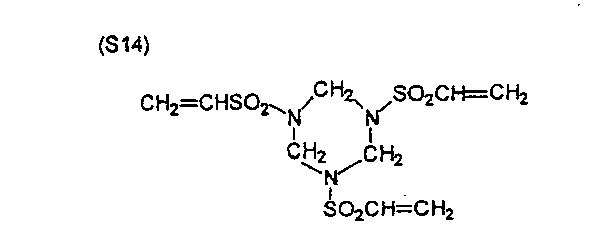

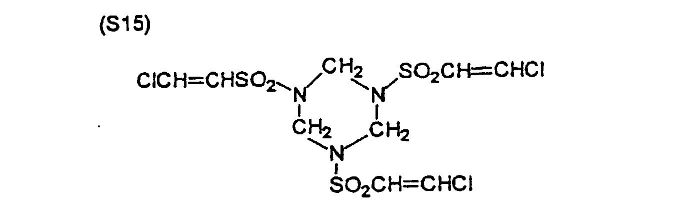

Representative examples of disulfone compound represented by the above-described formula (2) include 1,2-bis(vinylsulfonylacetamide)ethane [corresponding to the above-described S1].

As for a method for synthesizing a disulfone compound used in the present invention, the details have been described in a variety of gazettes, for example, such as Japanese Patent Publication No.S47-2429, Japanese Patent Publication No.S50-35807, Japanese Unexamined Patent Publication No.S49-24435, Japanese Unexamined Patent Publication No.S53-41551, Japanese Unexamined Patent Publication No.S59-18944 or the like.

In order to prepare a detection tool (in general, referred to as a DNA chip) for detecting by fixing a polynucleotide or an oligonucleotide originally from the nature such as DNA, RNA, DNA fragments, RNA fragments or the like by utilizing the reactive solid support obtained as described above, a method for bringing the above-described reactive solid support into contact with a nucleotide derivative or its analog equipped with a reactive group such as amino group which reacts with a vinyl sulfonyl group or its reactive precursor group on the above-described support surface to form a covalent bond is utilized. That is to say, in this way, a detection tool equipped with a probe molecule of the desired nucleotide derivative or its analog (what is called a DNA chip) can be prepared.

A vinyl sulfonyl group or its reactive precursor group bound via covalent bond to the surface of a solid phase substrate of the present invention can be readily preserved in a stable state since it has a high resistance to the hydrolysis, and can form a stable covalent bond by rapidly reacting with a nucleotide derivative or a reactive group of its analog in which amino group has been previously provided or a reactive group such as amino group has been introduced.

Representative examples of a nucleotide derivative and its analog used as a probe molecule include an oligonucleotide, a polynucleotide, and a peptide nucleic acid. These nucleotide derivatives or their analogs may be originally from the nature (DNA, DNA fragment, RNA or RNA fragment), or may be a synthetic compound. Moreover, nucleotide derivatives or its analog include a variety of analogous compounds such as what is called a LNA having a crosslinking group at its sugar unit portion (J. Am. Chem. Soc. 1998, 120:13252-13253) are included.

In the case where DNA fragments are used as a probe molecule, these are divided into two types depending on purposes. In order to examine the expression of a gene, it is preferable to use a polynucleotide such as cDNA, one portion of cDNA, or EST. Functions of these polynucleotides may be unknown, but in general, these are prepared by amplifying cDNA library, genomic library, or the total genome as a template on the basis of the sequences registered on a data base by a PCR method (hereinafter, referred to as "PCR product") . Ones not amplified by a PCR method can be also preferably used. In order to examine the mutation and polymorphism of a gene, it is preferable to synthesize a variety of oligonucleotides corresponding to mutation and polymorphism based on the known sequence which is to be the standard, and use them. Furthermore, in the case where the purpose is to analyze the base sequence, it is preferable to synthesize 4ⁿ (n represents the length of the base) species of oligonucleotides and use them. As for the base sequence of a DNA fragment, it is preferable that its sequence has been previously determined by a general base sequence determination method. The size of the DNA fragment is preferably from dimmer to 50-mer, and particularly preferably from 10- to 25-mer.

On one end of a nucleotide derivative such as an oligonucleotide and a DNA fragment, or its analog, a reactive group which forms a covalent bond by reacting with the foregoing vinyl sulfonyl group or its reactive precursor group is introduced. Such reactive groups include an amino group, an imino group, hydrazino group, carbomoyl group, hydrazinocarbonyl group or carboxyimido group, and the amino group is particularly preferable. The reactive group is usually bound to an oligonucleotide or a DNA fragment via a crosslinker. As the crosslinker, for example, an alkylene group or a N-alkylamino-alkylene group is utilized, and a hexylene group or a N-methylamino-hexylene group is preferable, and a hexylene group is particularly preferable. It should be noted that since a peptide nucleic acid (PNA) has an amino group, usually it is not necessary to introduce another reactive group.

Bringing a nucleotide derivative or its analog having a reactive group into contact with a reactive solid support is usually carried out by dotting the aqueous solution of the nucleotide derivative or its analog to the surface of the reactive solid support. Concretely, it is preferable that after an aqueous solution is prepared by dissolving or dispersing the nucleotide derivative or its analog having a reactive group in an aqueous medium, the aqueous solution is pipetted into 96 wells or 384 wells plastic plate, and the pipetted aqueous solution is dropped on the surface of the solid support using a spotter device or the like.

In order to prevent the nucleotide derivative or its analog from being dried after the dotting, a substance having a high boiling point may be added in the aqueous solution in which the nucleotide derivative or its analog is dissolved or dispersed. The substance having a high boiling point is preferably a substance that can be dissolved in the aqueous solution in which the nucleotide derivative or its analog to be dotted is dissolved or dispersed, does not hinder hybridization with a sample such as a nucleic acid fragment sample (target nucleic acid fragment) which is an obj ect of the detection, and has a not very high viscosity. Such substances include glycerin, ethylene glycol, dimethyl sulfoxide and a hydrophilic polymer having a lower molecular weight. Examples of the hydrophilic polymers include polyacrylamide, polyethylene glycol and sodium polyacrylate. Preferable molecular weight of this polymer is in the range from 10³ to 10⁶. As the substance having a high boiling point, it is more preferable to use glycerin or ethylene glycol, and particularly preferable to use glycerin. Preferable concentration of the substance having a high boiling point is in the range from 0.1 to 2% by volume, and particularly preferably 0.5 to 1% by volume in the aqueous solution of the nucleotide derivative or its analog.

Moreover, for the sake of the same purpose, it is also preferable to place the solid support after the dotting of a nucleotide derivative or its analog under the circumstances where the humidity is 90% or more and the temperature is in the range from 25 to 50 °C.

A post-treatment by ultraviolet ray, sodium borohydride or Schiff reagent may be carried out after the dotting of the nucleotide derivative or its analog having a reactive group. These post-treatments may be carried out by combining a plurality kinds of them, and the combination of heating treatment and the ultraviolet ray treatment is particularly preferable. These post-treatments are particularly effective in the case where the surface of the solid support is treated only by a polycationic compound. Incubation after the dotting is also preferable. After the incubation, removal of unreacted nucleotide derivatives or its analogs by washing is preferable.

A preferable fixed amount (numerical quantity) of nucleotide derivatives or its analogs with respect to the surface of the solid support is in the range from 10² to 10⁵ per cm². A preferable amount of nucleotide derivatives or its analogs is in the range from 1 to 10⁻¹⁵ mol, and is several ng or less in weight. By dotting, the aqueous solution of nucleotide derivatives or its analogs is fixed in a dot shape to the surface of the solid support. The shape of the dot is nearly circular. No variation in the shape is important for the quantitative analysis of a gene expression and the analysis of single nucleotide polymorphism. A preferable distance between each dot is in the range from 0 to 1.5mm, and a particularly preferable distance is in the range from 100 to 300 *µ*m. As for the size of a dot, a preferable diameter of it is in the range from 50 to 300 *µ*m. A preferable amount of the solution containing nucleotide derivatives or its analogs for dotting to the surface of the solid support is in the range from 100 pL to 1 µL, and a particularly preferable amount is in the range from 1 to 100 nL.

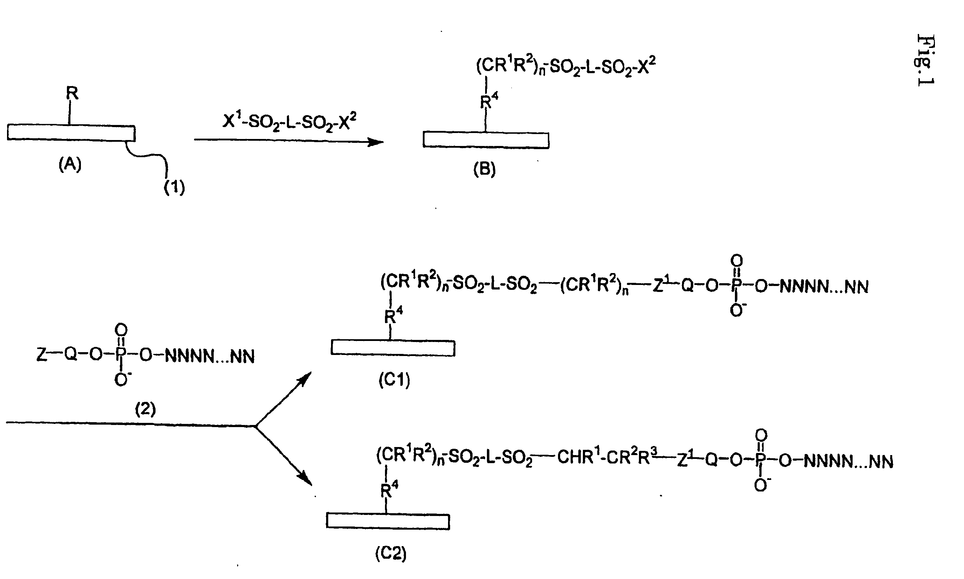

FIG. 1 schematically shows a method for manufacturing an oligonucleotide fixed solid support which is a representative aspect of the present invention and a configuration of a representative oligonucleotide fixed solid support.

As a method for manufacturing a solid support having convex and concave portions of the present invention to which an oligonucleotide is fixed, in the case where a disulfone compound represented by the foregoing formula (2) is employed, four types of methods for manufacturing can be utilized depending on X¹ and X²

FIG. 1 shows (a) a method for manufacturing a solid support (C1) to which the oligonucleotide is fixed using a disulfone compound of the formula (2) of which both of X¹ and X² represent -CHR¹-CR²R³Y (reactive precursor group), and (b) a method for manufacturing an oligonucleotide fixed solid support (C2) using the disulfone wherein X¹ represents -CHR¹-CR²R³Y and X² represents -CR¹=CR²R³. Here, supposing that X¹ represents a group reacting in the first place with a reactive group (R) introduced on the surface of the solid support 1, a method for manufacturing the solid support using the disulfone where X¹ represent -CR¹=CR²R³ may also be useful. Hereinafter, description will be made supposing that "X¹" is a group reacting in the first place with a reactive group (R) introduced on the surface of the solid support 1.

The manufacturing methods (a) and (b) will be described below.

Step (1) : A-(CR¹R²)ₙ-SO2-L-SO2-X² group is introduced to the solid support by bringing a disulfone compound represented by the formula (1) into contact with the surface of the solid support 1 into which the reactive group (R) is introduced [solid support (A)], and substituting -Y portion of X1 with a reactive group (R).

Step (2): A reactive group (Z) is added by bringing an oligonucleotide 2 having a reactive group (Z) at one end into contact with X² of -(CR¹R²)ₙ-SO₂-L-SO₂-X² group introduced in the Step (1), or -Y of the X² is substituted with the reactive group (Z) by bringing the oligonucleotide 2 into contact with Y.

A support of the present invention for fixing a probe molecule may be manufactured by the following method using -CR¹=CR²R³ as X¹, supposing that X¹ is a group reacting in the first place with the reactive group (R) introduced to the surface of the solid support 1.

Step (1): A -R³R²C-R¹HC-SO₂-L-SO₂-X² group is introduced to the surface of the solid support by bringing a disulfone compound represented by the formula (I) into contact with the surface of the solid support (A) consisted of solid support having the convex and concave portions (solid support 1) to the surface of which the active group (R) is introduced, and adding the reactive group (R) to -CR¹=CR²R³ of X¹.

Step (2) : The reactive group (Z) is added by bringing an oligonucleotide having the reactive group (Z) at one end into contact with X2 of -R³R²C-R₁HC-SO₂-L-SO₂-X₂ group introduced in the Step (1), or -Y of the X₂ is substituted with the reactive group (Z) by bringing the oligonucleotide 2 into contact with Y.

The oligonucleotide having the reactive group (Z) at one end is a compound shown by the reference numeral 2 in FIG. 1. A crosslinker (Q), though it is not essential, exists in general between the reactive group (Z) and phosphate ester group as a matter of convenience for preparation. A -phosphate group-NNNN,,,NN represents an oligonucleotide. R⁴ represents a group determined by the reaction between the reactive group (R) and X¹, and Z¹ represents a group determined by the reaction between X² and the reactive group (Z), respectively.

When the oligonucleotide 2 having the reactive group (Z) is dotted to the surface of the solid support (B) having the convex and concave portions shown in FIG. 1, although the reaction between X² or -Y of X² and the reactive oligonucleotide fragment 2 is occurred, an unreacted X² to which the oligonucleotide 2 is not bound also exists on the surface of solid support (B). In this case, there is a possibility that such X² reacts in a non-specific manner with a labeled nucleic acid fragment sample in a hybridization to be performed later resulting in a problem that non-specific binding may be measured. Therefore, it is preferable that the X² (i.e., a halogen atom of X², for example) has been previously subjected to a masking treatment. It is preferable that the masking treatment is performed by bringing an anionic compound having an amino group or a mercapto group into contact with the surface of the solid support (C) (or (C2)). Since the oligonucleotide 2 has a negative charge, the oligonucleotide 2 can be prevented from reacting with the unreacted X² by generating negative charge also on the surface of the solid support (C). As for such an anionic compound, any one can be used if it reacts with a halogen atom of X² and has a negative charge (COO⁻, SO₃⁻, OSO₃⁻, PO₃⁻, or PO₂⁻). Among them, an amino acid is preferable, and glycine or cysteine is particularly preferable. Further, taurine is also.preferably used.

The shelf life of a solid support manufactured according to the present invention, in which a nucleotide derivative or its analog is fixed, is usually several weeks for a cDNA fixed solid support to which cDNA is fixed, and the shelf life of a solid support to which a synthesized oligonucleotide is fixed is further longer. A solid support of the present invention to which an oligonucleotide or its analog is fixed is utilized for monitoring the gene expressions, determining the base sequences, analyzing the mutations, analyzing the polymorphism or the like. The principle of the detection is based on the hybridization with the labeled sample nucleic acid fragment, which will be described later.

As a labeling method, an RI method and a non-RI method (fluorescence method, biotin method, chemiluminescence method or the like) are known, and in the present invention, use of the fluorescence method is preferable. As for a fluorescent substance utilized for a fluorescent labeling, although any can be used if it can bind to the basic portion of nucleic acid. For example, a cyanine dye (e.g., Cy3, Cy5 or the like of Cy Dye™ series, which is commercially available), rhodamine 6G reagent, N-acetoxy-N2-acetylaminofluorene (AAF) or AAIF (iodine derivative of AAF) can be used.

As a nucleic acid fragment sample, usually, a nucleic acid fragment sample such as a DNA fragment sample or a RNA fragment sample whose sequence and function is not known is used.

It is preferable that a nucleic acid fragment sample is isolated from the cell or tissue sample of eucaryote for the purpose of examining the gene expression. In the case where the sample is a genome, it is preferably isolated from any given tissue sample except for red blood cell. It is preferable that any given tissue except for red blood cell is peripheral blood lymphocyte, skin, hair, sperm or the like. In the case where the sample is an mRNA, it is preferable that it is extracted from the tissue sample in which the MRNA is expressed. It is preferable that a labeled cDNA is prepared from mRNA by incorporating a labeled dNTP ("dNTP" means a deoxyribonucleotide in which the base is adenine (A), cytosine (C), guanine (G) or thymine (T)) using reverse transcription reaction. As a dNTP, use of dCTP is preferable because of chemical stability. Although the amount of the mRNA required for one hybridization is different depending on the amount of liquid to be spotted, and the type of labeling material, it is several µg or less. It is desired that the nucleic acid fragment sample has been previously depolymerized in the case where a DNA fragment on the nucleotide derivative or its analog fixed solid support is an oligoDNA. In the case of a prokaryotic cell, since the selective extraction of an mRNA is difficult, it is preferable that the total RNA is labeled.

As for a nucleic acid fragment sample, for the purpose of examining the mutation and polymorphism of a gene, it is preferable to obtain it by performing PCR of the target region in a reaction system containing the labeled primer or the labeled dNTP.

It is preferable that hybridization is carried out by dotting an aqueous solution, which is previously pipetted into a 96 wells or 384 wells plastic plate, in which labeled nucleic acid fragment samples are dissolved or dispersed, to the solid support of the present invention to which nucleotide derivatives or its analogs are fixed. The preferable amount of the solution for dotting is in the range from 1 to 100 nL. The hybridization is preferably carried out in the temperature range from room temperature to 70°C, and for the period from 6 to 20 hours. After the completion of hybridization, it is preferable that washing are performed using a mixed solution of a surfactant and a buffer solution to remove unreacted nucleic acid fragment samples. As a surfactant, it is preferable to use sodium dodecyl sulfate (SDS). As a buffer solution, citrate buffer solution, phosphate buffer solution, borate buffer solution, Tris buffer solution, Good' buffer solution or the like can be used. It is particularly preferable to use citrate buffer solution.

The hybridization using the solid support to which nucleotide derivatives or its analogs are fixed is characterized in that the amount of usage of the labeled nucleic acid fragment samples can be decreased to a very minute amount. Therefore, it is necessary to set the optimal conditions of the hybridization depending on the length of chain of the nucleotide derivatives or its analogs fixed to the solid support and the types of the labeled nucleic acid fragment samples. For the analysis of gene expression, it is preferable that a hybridization for a long time period is performed so as to be capable of sufficiently detecting even a lower expressing gene. For the detection of a single nucleotide polymorphism, it is preferable that a hybridization for a short period is performed. Moreover, it is also characterized in that comparison or quantitative determination of the expression amount are made possible using a single solid support to which DNA fragments are fixed by previously preparing two types of the nucleic acid fragment sample labeled by fluorescent substances different from each other and using them in a hybridization at the same time.

Furthermore, the present invention relates to a biological material chip characterized in that the residue A of at least one protein or protein binding substance is bound to the solid support having the convex and concave portions by covalent bond via sulfonyl group as is shown in the following formula (I).

Solid phase support-L-SO₂-X-A (I)

[In the above-described formula, L represents a linking group; X represents -CR¹ (R²) -CR³ (R⁴) -; each of R¹, R², R³ and R⁴ represents, independently from each other, a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of a protein or a protein binding substance (except for nucleic acid)]

Representative examples of a protein or a protein binding substance to be fixed include an antibody or antibody fragment, a ligand, an antigen, an antigenic determinant such as a hapten or the like, a receptor, a ligand, avidins and a nucleic acid recognition protein, but are not limited thereto. Representative examples of a nucleic acid recognition protein include double strand recognition protein.

Avidins include avidin, streptavidin and these altered bodies that can form a stable complex with biotin. The phrase "can form a stable complex" means that a complex having a dissociation constant approximate to the dissociation constant of biotin-avidin complex (10⁻¹⁵M) can be formed. The altered body means a body being modified or its fragment of avidin or streptavidin originally from the nature, or recombinants of these.

The nucleic acid recognition proteins include a double stranded DNA recognition substance, but are not limited thereto. The double stranded DNA recognition substances include a substance which recognizes a double stranded DNA and specifically binds to it. Examples of the double stranded DNA recognition substance include a DNA transcription factor, a mismatch repairing protein, a double stranded DNA recognition antibody, or a peptide nucleic acid. Furthermore, the double stranded DNA recognition substances include substances having Zinc Finger motif or Ring finger motif.

The DNA transcription factor is a substance that binds to the promoter region on gene and controls the transcription from DNA to mRNA (Takaaki, Tamura: Transcription Factor, published by Yodosha, Co. , Ltd., 1995) . Accordingly, it is known that the transcription factor specifically binds to a double stranded DNA of the specific sequence.

Among a large number of transcription factors, Zinc Finger Protein, that is, a transcription factor group having Zinc finger and Ring-Finger motifs, shows a very high occurrence rate in eucaryote, and 1% of the genome seems to code for them. Plabo et al. have analyzed the tertiary structure of Zinc Finger motif and elucidated the mechanism of its binding to DNA (Science 252: 809 (1991)). Further, Choo et al. have succeeded in preparing a Zinc Finger Protein group which binds to the specific sequence.but which does not exist in the nature, by a gene recombinant method (Nature 372: 642 (1994), PNAS 91: 11163 (1994)). Furthermore, the Scripps Research Institute group has succeeded in preparing a novel Zinc Finger Protein group by Phage Display (PNAS 95: 2812 (1998) ; 96: 2758 (1999)). As described above, the DNA transcription factor group represented by Zinc Finger Protein originally has the nature of binding to a double stranded DNA, and according to the researches in recent years, it has been made possible to prepare a recombinant which recognizes a given DNA sequence. It is possible to efficiently capture a double stranded DNA on a support by fixing such proteins.

Besides these, the nucleic acid binding substances include a helix-loop-helix protein and a substance having an Ets domain.

The protein binding substances to be fixed (except for nucleic acid) include hapten, biotins (biotin, biocytin, desthiobiotin, oxybiotin or their derivatives that can form a stable complex with avidin). The phrase "can form a stable complex" means that a complex having a dissociation constant approximate to the dissociation constant of biotin-avidin complex (10⁻¹⁵M) can be formed. Furthermore, peptides, sugars, hormones, pharmaceuticals, antibiotics and the like are listed.

A protein or a protein binding substance to be fixed to the solid support having the convex and concave portions forms a covalent bond via sulfonyl group with its amino group or mercapto group existing within it. Further, the covalent bond via sulfonyl group may be formed by introducing an amino group, an imino group, a hydrazine group, a carbomoyl group, a hydrazinocarbonyl group, a mercapto group or a carboxyimido group and the like.

A chip consisted of the solid support having the convex and concave portions to which a protein or a protein binding substance (e.g., antibody, avidins and nucleic acid recognition protein) is fixed, can fix the target substance on it, by bringing the chip into contact with a target substance (e.g., ligand, biotins and nucleic acid) that specifically react with the fixed protein or protein binding substance in the presence of an aqueous medium. It is desirable that a detectable label (e.g., fluorescene label, enzyme label or the like) is bound to the target substance of specific binding character to be fixed (e.g., ligand, biotins, and nucleic acid) so as to be capable of detecting its fixation from the exterior.

As for a solid support, any form, for example, a plate, a microwell, beads, stick or the like can be employed if it does not have an adverse effect on the binding formation between members of the specific binding partners. As the solid support, supports described above in the present specification can be used. For the convenience of operation, the solid support may be processed in a form of magnetic material or an electrode. The substance having the convex and concave properties is also as described above in the present specification.

In the present invention, a protein or protein binding substance A on the chip is bound to the solid support having the convex and concave portions by covalent bond via sulfonyl group as shown in the following formula (I):

Solid phase support-L-SO2-X-A (I)

In the formula (I), L represents a linking group; X represents -CR¹ (R²) -CR³ (R⁴) -; each of R¹, R², R³ and R⁴ represents independently from each other, a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and A represents a residue of a protein or a protein binding substance (except for nucleic acid).

In the formula (I), L represents a bivalent or more linking group for binding -SO2-X-A and the solid support. Examples of a -L- include any linking group selected from aliphatic, aromatic or heterocyclic compounds, hydro carbon chains that may be interrupted by a hetero atom, or combinations of them. Furthermore, L may be a singl bond.

In the formula (I), examples of an alkyl group having 1 to 6 carbon atoms include a methyl group, an ethyl group, a n-propyl group, a n-butyl group, and a n-hexyl group, and the methyl group is preferable. Examples of an aryl group having 6 to 20 carbon atoms include a phenyl group and a naphthyl group. It is preferable that each of R1, R2 and R3 represents a hydrogen atom.

Examples of an aralkyl group having 7 to 26 carbon atoms in total containing an alkyl chain having 1 to 6 carbon atoms include the combination of the examples of an alkyl group having 1 to 6 carbon atoms and the examples of an aryl group having 6 to 20 carbon atoms.

Furthermore, the present invention relates to a method for detecting a target substance comprising the steps of:
bringing the chip in accordance with the present invention described above into contact with a specimen containing a target substance that specifically binds to a protein or a protein binding substance supported on the surface of the chip; and
detecting formation of reciprocal binding between the protein or protein binding substance and the target substance.

The type of "specimen" referred to in the present invention is not particularly limited, and includes, for example, a blood such as peripheral venous blood, white blood cell, serum, urine, stool, sperm, saliva, a cultured cell, a tissue cell such as a variety of cells of organs, and any other sample containing nucleic acid. As for the specimen, the sample such as the tissue cell as described above may be used as it is. However, preferably, nucleic acid, ligand or the like which has been released by destructing the cell in the specimen sample is used as a specimen. The destruction of the cell in the specimen sample can be performed according to the conventional manner. For example, it can be performed by adding a physical action such as shaking, supersonic treatment from the exterior. The nucleic acid also can be released from the cell using a nucleic acid extraction solution (e.g., solution containing a surfactant such as SDS, Triton-X, Tween-20 or the like, or saponin, EDTA, protease or the like, or the like) . In the case where the nucleic acid is eluted using a nucleic acid extraction solution, the reaction can be promoted by incubation at the temperature of 37°C or higher.

Furthermore, the present invention relates to a method for manufacturing the chip according to the present invention comprising the step of contacting the solid support having the convex and concave portions that contains a vinyl sulfonyl group or its reactive precursor group represented by the following formula (II) on the surface, with at least one protein or protein binding substance having a reactive group which forms a covalent bond by reacting with the vinyl sulfonyl group or its reactive precursor group.

-L-SO₂-X' (II)

[In the formula (II), L represents a linking group for binding -SO₂-X' and the solid support; X' represents -CR¹=CR²(R³) or -CH (R¹) -CR² (R³) (Y) ; each of R¹, R² and R³ represents independently from each other, a hydrogen atom, an alkyl group having 1 to 6 carbon atoms, an aryl group having 6 to 20 carbon atoms, or an aralkyl group having 7 to 26 carbon atoms in total containing an alkyl chain having 1 to 6 carbon atoms; and Y represents a group substituted by a nucleophilic reagent, or a group which is eliminated as "HY" by base]

In the formula (II), examples of an alkyl group having 1 to 6 carbon atoms include a methyl group, an ethyl group, a n-propyl group, a n-butyl group, and a n-hexyl group, and the methyl group is particularly preferable. Examples of an aryl group having 6 to 20 carbon atoms include a phenyl group and naphthyl group. It is preferable that each of R¹, R² and R³ represents a hydrogen atom.

Examples of an aralkyl group having 7 to 26 carbon atoms in total containing an alkyl chain having 1 to 6 carbon atoms include the combination of the examples of an alkyl group having 1 to 6 carbon atoms and the examples of an aryl group having 6 to 20 carbon atoms.

In the formula (II), Y represents a group substituted by a nucleophilic reagent such as -OH, -OR⁰, -SH, NH₃, NH₂R⁰ (R⁰ represents a group such as alkyl group or the like except for hydrogen atom), or a group which is eliminated as "HY" by base. Examples thereof include a halogen atom, -OSO₂R¹¹, -OCOR¹², -OSO₃M, or a quaternary pyridinium group (R¹¹ represents an alkyl group having 1 to 6 carbon atoms, an aryl group having 6 to 20 carbon atoms, or an aralkyl group having 7 to 26 carbon atoms in total containing an alkyl chain having 1 to 6 carbon atoms; R¹² represents an alkyl group having 1 to 6 carbon atoms or halogenated alkyl group having 1 to 6 carbon atoms; M represents a hydrogen atom, an alkali metal atom, or an ammonium group).

An alkyl group of R¹¹, an aryl group of R¹¹ and an aralkyl group of R¹¹ may have a substituent. Examples of such a substituent include an atom or a group selected from the group consisted of a hydroxyl group, an alkoxy group having 1 to 6 carbon atoms, an alkenyl group having 1 to 6 carbon atoms, a carbamoyl group having 2 to 7 carbon atoms, an alkyl group having 1 to 6 carbon atoms, an aralkyl group having 7 to 16 carbon atoms, an aryl group having 6 to 20 carbon atoms, an sulfamoyl group (or its sodium salt, potassium salt or the like), a sulfo group (or its sodium salt, potassium salt or the like), a carboxylic acid group (or its sodium salt, potassium salt or the like), a halogen atom, an alkenylene group having 1 to 6 carbon atoms, an arylene group having 6 to 20 carbon atoms, sulfonyl group and their combinations.

In the formula (II), L represents a bivalent or more linking group for linking -SO₂-X' group and the solid support. Examples of a -L- include any linking group selected from aliphatic, aromatic or heterocyclic compound and a hydrocarbon chain that may be interrupted by a hetero atom, and a linking group selected from their combinations. Further L may be a single bond.

Preferable examples of the above-described "-X"' group are shown thereinafter:

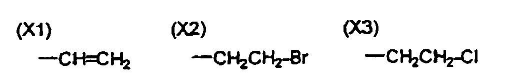

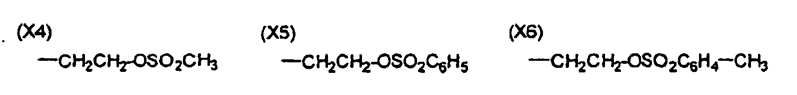

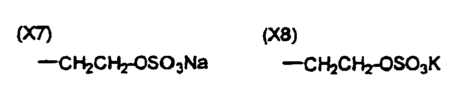

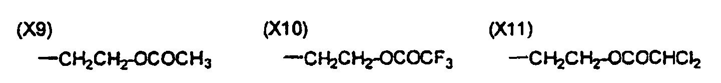

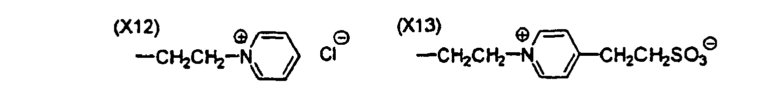

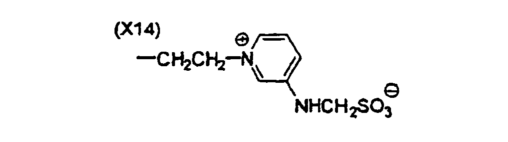

In the above-described concrete examples, it is preferable that "-X "' represents (X1), (X2), (X3), (X4), (X7), (X8), (X13) or (X14), and more preferable that "-X"' represents (X1) or (X2). And it is particularly preferable that "-X"' represents a vinyl group represented by (X1).

Since a covalent bond via a sulfonyl group utilized in the present invention has a high resistance to hydrolysis, it can be readily stored in a stable state, and can rapidly react with the protein in which an amino group or a mercapto group has been previously provided to form a stable covalent bond.

Usually, it is not necessary to introduce another reactive group, since the protein has an amino group or a mercapto group. However, since the tertiary structure of the protein largely participates in its function, in the case where the activity of the protein is lowered, it is preferable to introduce a reactive group at the specific position having no influence on the activity.

Dotting of the protein or protein binding substance is carried out by dotting an aqueous solution to the surface of the reactive solid support. Concretely, it is preferable to carry out the dotting along the following steps of: dissolving or dispersing the protein or protein binding substance into the aqueous medium to prepare an aqueous solution; pipetting the aqueous solution into a 96 wells or 384 wells plastic plate; dropping the aqueous solution thus pipetted to the surface of the solid support using a spotter device or the like. As is described above, although the spotter device can be used for dropping the protein or protein binding substance, there is a possibility of lowering the activity of the protein or protein binding substance depending on the property of a pin-head. In this case, it may be more preferable to use an ink jet device or the like depending on a case.

In order to prevent the protein or protein binding substance from being dried after the dotting of it, a substance having a high boiling point may be added in the aqueous solution. As a substance having a high boiling point, the substance that can be dissolved in the aqueous solution in which protein or protein binding substance to be dotted is dissolved or dispersed, does not hinder reaction with a sample which is an object of the detection, and has a not very high viscosity is preferably used. Examples of such substances include glycerin, ethylene glycol, dimethyl sulfoxide and a hydrophilic polymer having a lower molecular weight. The hydrophilic polymers include polyacrylamide, polyethylene glycol and sodium polyacrylate. Preferable molecular weight of this polymer is in the range from 10³ to 10⁶. As the substance having a high boilihg point, it is more preferable to use glycerin or ethylene glycol, and particularly preferable to use glycerin. Preferable concentration of the substance having a high boiling point can be adjusted depending on the activity of the protein after the dotting.

Moreover, for the sake of the same purpose, it is also preferable to place the solid support after the dotting of the protein or protein binding substance under the circumstances where the humidity is 90% or more and the temperature is in the range from 25 to 40°C.

A preferable fixed amount (numerical quantity) of the protein or protein binding substance with respect to the surface of the solid support is in the range from 1 to 10⁵ per cm². By the dotting, the aqueous solution of the protein or protein binding substance is fixed in a dot shape to the surface of the solid support. The shape of the dot is nearly circular. The distance between the respective dots, the size of the dot and the volume of the aqueous solution when it is dotted vary depending on its intended use.

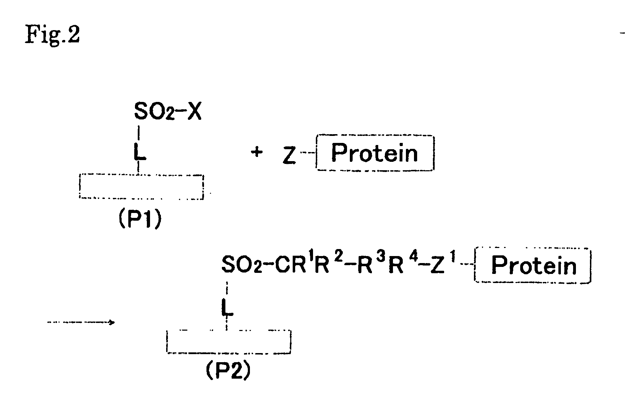

FIG. 2 schematically shows a configuration of a protein chip, which is the representative aspect of the present invention.

When the protein A having the reactive group (Z) is dotted to the surface of the solid support (P1) having the convex and concave portions shown in FIG. 2, the reaction between X and the protein occurs. However, an unreacted X to which the protein is not bound also exists on the surface of the solid support (P1). In this case, there is a possibility that such X reacts in a non-specific manner with a labeled ligand sample and the like in a reaction to be performed later, resulting in a problem that non-specific binding may be measured. Therefore, it is preferable that the X has been previously subjected to a blocking treatment. It is preferable that the blocking treatment is performed by bringing a compound having an amino group or a mercapto group into contact with the surface of the solid support (P2). In order to prevent the non-specifical binding of a ligand to be reacted, it is preferable to perform the blocking treatment using a protein blocking agent, specifically, BSA, casein, gelatin or the like. As the result of the blocking, BSA or the like exists on the surface of the solid support (P2) which has not been dotted, thereby preventing the binding of the ligand. Moreover, in the case where the nucleic acid is to be reacted, the blocking treatment can be carried out by contacting an anionic compound having an amino group or a mercapto group other than the above-described protein blocking agent. In the case where the substance with which the protein is reacted is a nucleic acid, since the nucleic acid has a negative charge, it can prevent the nucleic acid from reacting with an unreacted X by generating the negative charge also on the surface of the solid support (P2). As for such an anionic compound, any compound can be used if it reacts with X and has a negative charge (COO⁻, SO₃⁻, OSO₃⁻, PO₃⁻, or PO₂⁻). Among them, an amino acid is preferable, and glycine or cysteine is particularly preferable. Further, taurine is also preferably used.

A protein chip which is a representative aspect of the present invention is utilized for the analysis of protein interactions, the analysis of the protein expression and the drug development search. Furthermore, in the case where the protein is a nucleic acid binding protein, it can be utilized for the mutation analysis and the nucleotide polymorphism analysis depending on its recognition nucleic acid sequence.

The principle of the detection is based on the reaction with the labeled ligand or nucleic acid. As labeling methods, although a RI method and a non-RI method (fluorescence method, biotin method, chemiluminescence method or the like) are known, it is not particularly limited. For example, in the case of the fluorescence method, as a fluorescent substance utilized for a fluorescence labeling, any can be used if it can bind to the basic portion of nucleic acid or protein amino acid residue. For example, cyanine dye (e.g., Cy3, Cy5 or the like of Cy Dye™ series, which is commercially available), rhodamine 6G reagent, N-acetoxy-N2-acetylaminofluorene (AAF) or AAIF (iodine derivative of AAF) can be used.

In the case of a protein chip in which a nucleic acid recognition protein is fixed, it is preferable that the target nucleic acid in the specimen is directly detected without amplifying it by a PCR method or the like. However, it may be detected after it has been previously amplified. The target nucleic acid or its amplified body can be easily detected by previously labeling it. In order to label the nucleic acid, a method of using an enzyme (Reverse Transcriptase, DNA polymerase, RNA polymerase, Terminal deoxy trasferase or the like) is often used. Further, the labeling substance may be directly bound by chemical reaction. Such labeling method has been described in some books as the known technology (Shintaro Nomura: De-isotope Experiment Protocol 1, published by Shujun Sha, Co., Ltd., 1994; Shintaro Nomura: De-isotope Experiment Protocol 2, published by Shujun Sha, Co., Ltd., 1998; Masaaki Muramatsu: DNA Microarray and Advanced PCR Method Labeling Material, published by Shujun Sha, Co., Ltd., 2000). It is preferable that the labeling material is a material capable of making a detectable signal. In the case where the labeling material is a material having an amplifying ability of signal such as an enzyme and a catalyst, the detection sensitivity of DNA is largely enhanced.

However, since the labeling operation described above is generally troublesome, a method of measuring the nucleic acid in the specimen without previous labeling of the nucleic acid can be used as a more preferable method of detection. For the purpose of it, for example, a DNA intercalating agent which recognizes a double stranded DNA, that is, what is called a DNA intercalator can be used. By the use of a DNA intercalator, not only the operation of the detection is made easier, but also the sensitivity of the detection is enhanced. For example, in the case where a DNA of 1000 bp is to be detected, although a labeling method can introduce several labeling materials at most, in the case where the intercalator is used, 100 or more labeling materials can be introduced.

The DNA intercalator may be a material which can form a detectable signal in itself, or the signal formation material may be bound to the side chain of intercalator, or bound to the intercalator via a specific binding pair such as a biotin-avidin, an antigen-antibody or a hapten-antibody. It is preferable that the detectable signal used in the present invention is the signal detectable by a fluorescene detection, a luminescence detection, a chemiluminescence detection, a bioluminescence detection, an electrochemiluminescence detection, a radiation detection, an electrochemical detection or a colorimetric detection, but it is not limited to them.

In the case where a ligand is a target, there can be used a substance obtained by reacting a succinimide body of a cyanine dye (e.g., Cy3, Cy5 or the like of Cy Dye™ series, which is commercially available), rhodamine 6G reagent, N-acetoxy-N₂-acetylaminofluorene (AAF) or AAIF (iodine derivative of AAF) with an amino group existing inside.

The present invention will be more concretely described below by the following Examples, but the present invention is not limited by these Examples.

### Examples

### Example 1: Preparation of DNA fragment fixed slide, and measurement of fixed amount of DNA fragment

The preparation and its performance of a reactive solid support having the convex and concave properties, and the comparison with a reactive solid support not having the convex and concave properties as a comparative control are shown below.

### (1) Preparation of solid support (A) having convex and concave properties onto which vinyl sulfonyl group has been introduced

200 ml of the suspension of 5% by weight of a colloidal silica (Snowtechs PS-S [Nissan Chemical Industries Co., Ltd.] /average particle diameter about 10 nm) was prepared. In the suspension, 20 pieces of glass slides previously washed, being contained in a basket commercially available, were immersed for 30 seconds. After drawing up them, draining off was performed and dried for 10 minutes by a drier at 45°C. Next, each glass slide was reacted with 200 ml of 2% by weight solution of Shinetsu Silicone KBE 903 (Shinetsu Chemical Industry, Co., Ltd.) for 3 minutes using a commercially available slide washer. After the completion of the reaction, it was washed for 1 minute (using slide washer) with 200 ml of ultrapure water. While exchanging the ultrapure water, washings were repeated more two times under the above-described washing conditions. After the completion of washings, and after it had been dried for 10 minutes by the drier at 45°C, it was put into the oven set at 110°C was thermally treated for 10 minutes. After cooling it, 3% by weight of 1,2-bis (vinyl sulfonyl acetamide) ethane/pH 8.0 borate buffer solution was reacted for 120 minutes using the slide washer. After the completion of the reaction, a 20 second washing was repeated three times with ultrapure water. The drying was performed for 30 minutes by the drier set at 25°C to obtain a solid support (A) having the convex and concave properties. When the surface of (A) was observed using an atomic force microscope (AFM), it was confirmed that the thickness of the layer containing vinyl sulfonyl groups was about 10 nm, and the glass slide was coated with a single layer.

### (2) Dotting of DNA fragment and measurement of fluorescence intensity

The aqueous solution (1 x 10⁻⁶M, 1 µL) consisted of dispersion of the DNA fragment (3'-TCCTCCATGTCCGGGGAGGATCTGACACTTCAAGGTCTAG-5') SEQiDN02, of which 3' end and 5' end were modified with an amino group and a fluorescence labeling reagent (Fluoro Link Cy5-dCTP, made by Amersham Pharmacia Biotech, Co., Ltd.) respectively, in 0.1M carbonate buffer solution (pH 9.3), was dotted on the slide (A) obtained in the above-described (1). Immediately, the solid support after the dotting was left for 1 hour at the temperature of 25°C and humidity of 90%. The solid support was washed with the mixed solution of 0.1 wt% SDS (sodium dodecyl sulfate) and 2 x SSC (2 x SSC: solution in which the stock solution of SSC is diluted into 2-fold; SSC: standard salt-citric acid buffer solution) two times, and washed with 0.2 x SSC aqueous solution once. Then, the slide after the above-described washings was immersed in 0.1 M glycine Aqueous solution (pH 10) for 1 hour and 30 minutes, washed with distilled water, and dried at room temperature to obtain a solid support (B) to which DNA fragments were fixed. The fluorescence intensity of the surface of this solid support (B) measured by a fluorescence scanning device was 15,250.

### (3) Preparation of a comparative control slide, and its performances

The glass slide was reacted with 200 ml of 2% by weight solution of Shinetsu Silicone KBE 903 (Shinetsu Chemical Industry, Co., Ltd.) for 3 minutes using a commercially available slide washer. After the completion of the reaction, it was washed for 1 minute (using the slide washer) with 200 ml of ultrapure water. While exchanging the ultrapure water, washings were repeated more two times under the above-described washing conditions. After the completion of washings, it was dried for 10 minutes by the drier at 45°C, then put into the oven set at 110°C and thermally treated for 10 minutes. After cooling it, 3% by weight of 1,2-bis (vinyl sulfonyl acetamide) ethane/pH 8.0 borate buffer solution was reacted for 120 minutes using the slide washer. After the completion of the reaction, a 20 second washing was repeated three times with ultrapure water. The drying was performed for 30 minutes by the drier set at 25°C. Evaluation similar to the above-described (2) was performed, and 2300 of the fluorescence intensity was obtained.

The above-described results are summarized in the following Table.

**Table 1**

| | fluorescence intensity |
|---|---|
| solid support having convex and concave properties (Invention: Example 1 (1) and (2)) | 15250 |
| comparative control slide (Comparative: Example 1(3)) | 2300 |

As seen from the results of Table 1, it is understood that, according to the fixation method of the present invention, DNA fragments are efficiently fixed in a high density on the slide glass.

### Example 2: Detection of sample DNA fragment

### (1) Preparation of DNA fragment fixed solid support having convex and concave properties

The aqueous solution (1 x 10⁻⁶M, 1 µL) consisted of dispersion of 40 mer of the DNA fragment (3'-TCCTCCATGTCCGGGGAGGATCTGACACTTCAAGGTCTAG-5'), SEQiDN02, of which 3' end was modified with an amino group, in 0.1M carbonate buffer solution (pH 9.3), was dotted on the solid support having the convex and concave properties (A) obtained in the above-described (1) of Example 1. Immediately, the solid support after the dotting was left for 1 hour at the temperature of 25°C and humidity of 90%. The solid support was washed with the mixed solution of 0.1 wt% SDS (sodium dodecyl sulfate) and 2 x SSC (2 x SSC: solution in which the stock solution of SSC is diluted into 2-fold; SSC: standard salt-citric acid buffer solution) two times, and washed with 0.2 x SSC aqueous solution once. Then, the slide after the above-described washings was immersed in 0.1 M glycine aqueous solution (pH 10) for 1 hour and 30 minutes, washed with distilled water, dried at room temperature to obtain a solid support (C) to which DNA fragments were fixed.

### (2) Detection of sample DNA fragment

The aqueous solution consisted of dispersion of 22 mer of the sample oligonucleotide (CTAGTCTGTGAAGTTCCAGAIC-5'), SEQiDN01, of which 5' end was bound with Cy5, in the solution for hybridization (mixed solution of 4 x SSC and 10% by weight of SDS) (20 µL) was dotted on the solid support (C) obtained in the above-described (1). Then, the surface was protected by a cover glass for microscopy, and incubated at 60°C for 20 hours within a moisture chamber. After the incubation, it was washed with the mixed solution of 0.1% by weight SDS and 2 x SSC, the mixed solution of 0.1% by weight SDS and 0.2 x SSC, and 0.2 x SSC aqueous solution in turn, centrifuged at 600 rpm for 20 seconds, and dried at room temperature. Fluorescence intensity of the surface of the glass slide measured by a fluorescence scanning device was 10350.

On the other hand, when the detection of the sample DNA fragment was performed using the comparative control slide, the fluorescence intensity was 1500.

It is understood that a sample DNA fragment having the complementarity to the fixed DNA fragment can be defected in a high sensitivity by employing the DNA fragment fixed solid support having the convex and concave properties prepared according to a fixation method of the present invention.

### Example 3 : Preparation of a DNA fragment fixed slide, and measurement of fixed amount of DNA fragment

### (1) Preparation of a solid support having convex and concave properties to which vinyl sulfonyl group has been introduced

A coated slide glass for DNA microarray (Type 2 highly densified amino group introduced type [Matsunami Glass Industry, Co., Ltd.]) was reacted for 120 minutes with 3% by weight of 1,2-bis (vinyl sulfonyl acetamide) ethane/pH 8.0 borate buffer solution using the slide washer. After the completion of the reaction, a 20 second washing was repeated three times with ultrapure water. The drying was performed for 30 minutes by a drier set at 25°C . An evaluation was performed similarly to (2) of Example 1, and 3600 of the fluorescence intensity was obtained.

### Example 4: Detection of sample DNA fragment

### (1) Preparation of DNA fragment fixed support using the slide glass made by Matsunami Glass, Co., Ltd.

The aqueous solution (1 x 10⁻⁶M, 1 µL) consisted of dispersion of 40 mer of the DNA fragment (3'-TCCTCCATGTCCGGGGAGGATCTGACACTTCAAGGTCTAG-5') SEQiDN02, of which 3' end was modified with an amino group , in 0.1M carbonate buffer solution (pH 9.3) was dotted on the solid support having the convex and concave properties obtained in the above-described (1) of Example 3. Immediately, the solid support after the dotting was left for 1 hour at the temperature of 25 !!degree!! and humidity of 90%. The solid support was washed in turn with the mixed solution of 0.1 wt% SDS (sodium dodecyl sulfate) and 2 x SSC (2 x SSC: solution in which the stock solution of SSC is diluted into 2-fold; SSC: standard salt-citric acid buffer solution) two times, and washed with 0.2 x SSC aqueous solution once. Then, the slide after the above-described washings was immersed in 0.1 M glycine aqueous solution (pH 10) for 1 hour and 30 minutes, washed with distilled water, dried at room temperature to obtain a solid support (D) to which DNA fragments were fixed.

### (2) Detection of sample DNA fragment

The aqueous solution consisted of dispersion of 22 mer of the sample oligonucleotide (CTAGTCTGTGAAGTTCCAGATC-5'), SEQiDN01, of which 5' end was bound with Cy5, in the solution for hybridization (mixed solution of 4 x SSC and 10% by weight of SDS) (20µL) was dotted on the solid support (D) obtained in the above-described (1). Then, the surface was protected by a cover glass for microscopy, and incubated at 60°C for 20 hours within a moisture chamber. After the incubation, it was washed with the mixed solution of 0.1% by weight SDS and 2 x SSC, the mixed solution of 0.1% by weight SDS and 0.2 x SSC, and 0.2 x SSC aqueous solution in turn, centrifuged at 600 rpm for 20 seconds, and dried at room temperature. Fluorescence intensity of the surface of the glass slide measured by a fluorescence scanning device was 15360.

### Example 5: Preparation of DNA fragment fixation slide, and measurement of fixed amount of DNA fragment

The preparation and its performance of a reactive solid support having the convex and concave properties, and the comparison with a reactive solid support not having the convex and concave properties as a comparative control are shown below.

### (1) Preparation of solid support (A) having convex and concave properties to which vinyl sulfonyl group has been introduced

75 mg of polyvinyl alcohol was dissolved in 1000 ml of ultrapure water. After the dissolving, 500 µl of a silane coupling agent (KBE 903; made by Shinetsu Chemical Industry, Co., Ltd.) was added, and stirred for 10 minutes by a magnet stirrer. The glass substrate (76 mm x 26 mm) was immersed in the solution for 30 seconds, then drawn up. After its excessive liquid was drained off, it was dried by the drier at 45°C. Next, this glass substrate was heated in an oven set at 110°C for 10 minutes to fix the silane on the glass substrate. Further, after cooling the above-described glass substrate, it was reacted with 3% by weight of 1,2-bis (vinyl sulfonyl acetamide) ethane/pH 8.0 borate buffer solution for 120 minutes using the slide washer. After the completion of the reaction, a 20 second washing was carried out three times with ultrapure water. The drying was performed for 30 minutes by the drier set at 25°C .

### (2) Dotting of DNA fragment and measurement of fluorescence intensity

The aqueous solution (1 x 10⁻⁶M, 1 µL) consisted of dispersion of the DNA fragment (3'-TCCTCCATGTCCGGGGAGGFTCTGACACTTCAAGGTCTAG-5') SEQiDN02, of which 3' end and 5' end were modified with an amino group and a fluorescence labeling reagent (Fluoro Link Cy5-dCTP, made by Amersham Pharmacia Biotech, Co., Ltd.) respectively, in 0.1M carbonate buffer solution (pH 9.3) was dotted on the slide (A) obtained in the above-described (1). Immediately, the solid support after the dotting was left for 1 hour at the temperature of 25°C and humidity of 90%. The solid support was washed in turn with the mixed solution of 0.1 wt% SDS (sodium dodecyl sulfate) and 2 x SSC (2 x SSC: solution in which the stock solution of SSC is diluted into 2-fold; SSC: standard salt-citric acid buffer solution) two times, and washed with 0.2 x SSC aqueous solution once. Then, the slide after the above-described washings was immersed in 0.1 M glycine aqueous solution (pH 10) for 1 hour and 30 minutes, washed with distilled water, dried at room temperature to obtain a solid support (B) to which DNA fragments were fixed. The fluorescence intensity of the surface of this solid support (B) measured by a fluorescence scanning device was 22,250.

### (3) Preparation of comparative control slides and their performances

The glass slide was reacted with 200 ml of 2% by weight solution of Shinetsu Silicone KBE 903 (Shinetsu Chemical Industry, Co., Ltd.) for 3 minutes using a commercially available slide washer. After the completion of the reaction, it was washed for 1 minute (using the slide washer) with 200 ml of ultrapure water. While exchanging the ultrapure water, washings we=e repeated more two times under the above-described washing conditions. After the completion of washings, it was dried for 10 minutes by the drier at 45°C , then put into the oven set at 110°C and thermally treated for 10 minutes. After cooling it, 3% by weight of 1,2-bis (vinyl sulfonyl acetamide) ethane/pH 8.0 borate buffer solution was reacted for 120 minutes using the slide washer. After the completion of the reaction, a 20 second washing was repeated three times with ultrapure water. The drying was performed for 30 minutes by the drier set at 25°C. Evaluation similar to the above-described (2) was performed, and 1750 of the fluorescence intensity was obtained.

As seen from the above mentioned results , it is understood that, according to the fixation method of the present invention, DNA fragments are efficiently fixed in a high density on the slide glass.

### Example 6: Detection of sample DNA fragment

### (1) Preparation of DNA fragment fixed solid support having convex and concave properties

The aqueous solution (1 x 10⁻⁶M, 1 *µ*L) consisted of dispersion of 40 mer of the DNA. fragment (3'-TCCTCCATGTCCGGGGAGGATCTGACACTTCAAGGTCTAG-5'), SEQiDN02, of which 3' end was modified with an amino group , in 0.1M carbonate buffer solution (pH 9.3) was dotted on the solid support having the convex and concave properties (A) obtained in the above-described (1) of Example 5. Immediately, the solid support after the dotting was left for 1 hour at the temperature of 25°C and humidity of 90%. The solid support was washed in turn with the mixed solution of 0.1 wt% SDS (sodium dodecyl sulfate) and 2 x SSC (2 x SSC: solution in which the stock solution of SSC is diluted into 2-fold; SSC: standard salt-citric acid buffer solution) two times, and washed with 0.2 x SSC aqueous solution once. Then, the slide after the above-described washings was immersed in 0.1 M glycine aqueous solution (pH 10) for 1 hour and 30 minutes, washed with distilled water, dried at room temperature to obtain a solid support (C) to which DNA fragments were fixed.

### (2) Detection of sample DNA fragment

The aqueous solution consisted of dispersion of 22 mer of the sample oligonucleotide (CTAGTCTGTGAAGTTCCAGATC-5'), SEQiDN01, of which 5' end was bound with Cy5, in the solution for hybridization (mixed solution of 4 x SSC and 10 % by weight of SDS) (20 *µ*L) was dotted on the solid support (C) obtained in the above-described (1) . Then, the surface was protected by a cover glass for microscopy, and incubated at 60°C for 20 hours within a moisture chamber. After the incubation, it was washed with the mixed solution of 0.1% by weight SDS and 2 x SSC, the mixed solution of 0.1% by weight SDS and 0.2 x SSC, and 0.2 x SSC aqueous solution in turn, centrifuged at 600 rpm for 20 seconds, and dried at room temperature. Fluorescence intensity of the surface of the glass slide measured by a fluorescence scanning device was 18400.

On the other hand, when the detection of the sample DNA fragment was performed using the comparative control slide, the fluorescence intensity was 1500.

It is understood that a sample DNA fragment having the complementarity to the fixed DNA fragment can be detected in a high sensitivity by employing the DNA fragment fixed solid support having the convex and concave properties prepared according to the present invention.

### Example 7: Detection of ligand by antibody fixed slide

### (1) Preparation of solid support having convex and concave properties (A) to which vinyl sulfonyl group has been introduced

200 ml of the suspension of 5% by weight of a colloidal silica (Snowtechs PS-S[Nissan Chemical Industries Co., Ltd.]/average particle diameter about 10 nm) was prepared. In the suspension, 20 pieces of glass slides previously washed, being contained in a basket commercially available, were immersed for 30 seconds. After drawing up them, draining off was performed and dried for 10 minutes by a drier at 45°C. Next, each glass slide was reacted with 200 ml of 2% by weight solution of Shinetsu Silicone KBE 903 (Shinetsu Chemical Industry, Co., Ltd.) for 3 minutes using a commercially available slide washer. After the completion of the reaction, it was washed for 1 minute (using slide washer) with 200 ml of ultrapure water. While exchanging the ultrapure water, washings were repeated more two times under the above-described washing conditions. After the completion of washings, and after it had been dried for 10 minutes by the drier at 45°C, it was put into the oven set at 110°C, and was thermally treated for 10 minutes. After cooling it, 3% by weight of 1,2-bis (vinyl sulfonyl acetamide) ethane/pH 8.0 borate buffer solution was reacted for 120 minutes using the slide washer. After the completion of the reaction, a 20 second washing was repeated three times with ultrapure water. The drying was performed for 30 minutes by the drier set at 25°C to obtain a solid support (A) having the convex and concave properties. When the surface of (A) was observed using an atomic force microscope (AFM), it was confirmed that the thickness of the layer was about 10 nm, and the glass slide was coated with a single layer.

### (2) Fixation of antibody

Goat Anti-Human IgG (Jackson ImmunoResearch) was diluted with PBS (100, 20, 4, 0.8, 0.16 ng/*µ*L, 1/*µ*L), and dotted on the solid support (A) prepared in the above-described (1). Immediately, the solid support after the dotting was left at 25°C for 3 hours in a saturated common salt chamber. Then the blocking treatment was performed by immersing it in 1% BSA/0.05% Tween 20-PBS (PBS-T) for 1 hour to obtain an antibody slide (B).

### (3) Reaction with ligand and detection of it

HybriWell (Grace Bio-Labs) was intimately contacted to the antibody slide (B) prepared in the above-described (2). Human IgG-Cy5 (Jackson ImmunoResearch) was diluted with 1% BSA/PBS-T into 2 *µ*g/ml. After 100 *µ*L of the diluted solution was added within HybriWell, it was incubated at 25 !! degree !! for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, it was 39.2 at the position where the antibody was spotted with 100 ng/µL, indicating a large increase from a background fluorescence intensity. Therefore, it is understood that by employing the antibody fixation solid support of the present invention consisted of the solid support having the convex and concave properties to which antibodies have been bound via sulfonyl group, a ligand having reactivity with the antibody fixed on the antibody fixation solid support can be efficiently detected.

### Example 8: Detection of ligand by antibody fixed slide

### (1) Fixation of antibody

Rabbit Anti Streptavidin (Polysienece) was diluted with PBS (100 ng/µL), and each 10 µL of it was pipetted into 384 wells plate. It was dotted on the solid support (A) prepared in the above-described Example 7 (1) using an arrayer made by Cartecyan (PixSys 5500). Immediately, the solid support after the dotting was left at 25°C for 3 hours in a saturated common salt chamber, then the blocking treatment was performed by immersing it in 1/4 x Block Ace (Dainippon Pharmaceuticals)/0.05% Tween 20-PBS (PBS-T) for 1 hour to obtain an antibody slide (C).

### (2) Labeling of ligand

1 mg of Streptavidin (Wako Junyaku) was dissolved in 400 µl of 0.1 M NaHCO₃ (pH 8.0). It was transferred to a Cy3-monofunctional tube (Amersham Pharmacia Biotech) and incubated at room temperature for 30 minutes. 100 µl of 1 M Tris/HCl (pH 8.0) was added to it, and further incubated at room temperature for 30 minutes to stop the reaction. The reacted solution was purified by NAP-5 column (Amersham Pharmacia Biotech), and Cy3-labeled Streptavidin was obtained.

### (3) Reaction with ligand and detection of it

SecureSeal Hybridization Chamber (SA 500, Grace Bio-Labs) was intimately contacted to the antibody slide (C) prepared in the above-described (1) . Cy3-labeled Streptavidin obtained in the above-described (2) was diluted with 1/4 x Block Ace/PBS-T into 2 *µ*g/ml. After 500 *µ* L of it was added within SA 500, it was incubated at 25°C for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, it was 19,320, indicating a large increase from the background fluorescence intensity. Therefore, it is understood that by employing the antibody fixation solid support of the present invention consisted of the solid support having the convex and concave properties to which antibodies have been bound via sulfonyl group, a ligand having reactivity with the antibody fixed on the antibody fixed solid support can be efficiently detected also in a microarray system.

### Example 9: Detection of ligand by antibody fixed slide

### (1) Preparation of solid support (D) to which vinyl sulfonyl group has been introduced

20 pieces of coated slide glass for DNA microarray (Type 2 highly densified amino group introduced type [Matsunami Glass Industry, Co., Ltd.]) contained in a commercially available basket, were reacted for 120 minutes with 3% by weight of 1,2-bis (vinyl sulfonyl acetamide) ethane/pH 8.0 borate buffer solution using the slide washer. After the completion of the reaction, a 20 second washing was repeated three times with ultrapure water. The drying was performed for 30 minutes by a drier set at 25°C.

### (2) Fixation of antibody

Goat Anti-Human IgG (Jackson ImmunoResearch) was diluted with PBS (100 ng/*µ*L), and each 10 *µ*L of it was pipetted into 384 wells plate. It was dotted on the solid support (D) prepared in the above-described Example 9 (1) using an arrayer made by Cartecyan (PixSys 5500). Immediately, the solid support after the dotting was left at 25°C for 1 hour in a saturated common salt chamber, then the blocking treatment was performed by immersing it in 1/4 x Block Ace (Dainippon Pharmaceuticals)/0.05% Tween 20-PBS (PBS-T) for 1 hour to obtain an antibody slide (E).

### (3) Reaction with ligand and detection of it

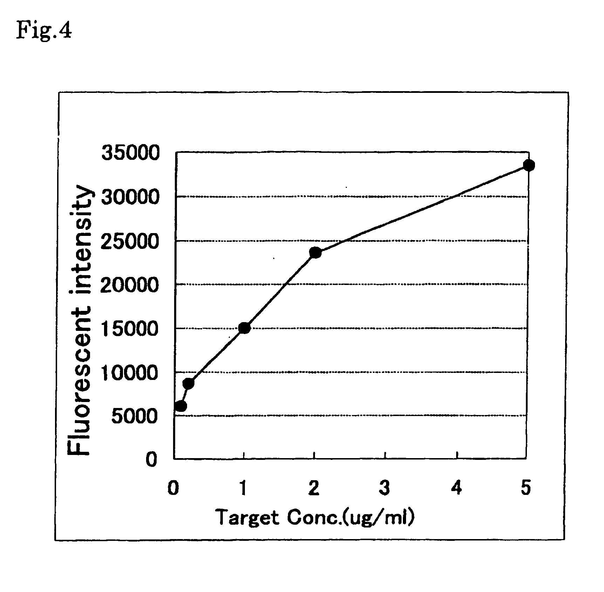

SecureSeal Hybridization Chamber (SA 500, Grace Bio-Labs) was intimately contacted to the antibody slide (E) prepared in the above-described (1). Human IgG-Cy5 (Jackson ImmunoResearch) was diluted with 1/4 x Block Ace/PBS-T into 5, 2, 1, 0.2, 0.1 *µ*g/ml. After 500 *µ*L of each diluted solution was added within SA500, it was incubated at 25°C for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, they were 33525, 23629, 15001, 8667 and 6053, respectively, on the slides to which 5, 2, 1, 0.2, 0.1 µg/ml of Human IgG-Cy5 were added. And approximate linearity was confirmed in the range from 0.2 to 2 µg/ml (FIG. 4).

### Example 10: Detection of ligand by antibody fixed slide

### (1) Preparation of solid support (A) having convex and concave properties to which vinyl sulfonyl group has been introduced

75 mg of polyvinyl alcohol was dissolved in 1000 ml of ultrapure water. After the dissolving, 500 µl of a silane coupling agent (KBE 903; made by Shinetsu Chemical Industry, Co., Ltd.) was added, and stirred for 10 minutes by a magnetic stirrer. The glass substrate (76 mm x 26 mm) was immersed in the solution for 30 seconds, then drawn up. After its excessive liquid was drained off, it was dried by the drier at 45°C. Next, this glass substrate was heated in an oven set at 110°C for 10 minutes to fix the silane on the glass substrate.

Further, after cooling the above-described glass substrate, it was reacted with 3% by weight of 1,2-bis (vinyl sulfonyl acetamide) ethane/pH 8.0 borate buffer solution for 120 minutes using the slide washer. After the completion of the reaction, a 20 second washing was carried our three times with ultrapure water. The drying was performed for 30 minutes by the drier set at 25°C to obtain the solid support having the convex and concave properties (A).

### (2) Fixation of antibody

Goat Anti-Human IgG (Jackson ImmunoResearch) was diluted with PBS (100, 20, 4, 0.8, 0.16 ng/*µ*L, 1 µL), and dotted on the solid support (A) prepared in the above-described (1). Immediately, the solid support after the dotting was left at 25°C for 3 hours in a saturated common salt chamber. Then the blocking treatment was performed by immersing it in 1% BSA/0.05% Tween 20-PBS (PBS-T) for 1 hour to obtain an antibody slide (B).

### (3) Reaction with ligand and detection of it

HybriWell (Grace Bio-Labs) was intimately contacted to the antibody slide (B) prepared in the above-described (2). Human IgG-Cy5 (Jackson ImmunoResearch) was diluted with 1% BSA/PBS-T into 2µg/ml. After 100µL of the diluted solution was added within HybriWell, it was incubated at 25°C for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, it was 40.2 at the position where the antibody was spotted at 100 ng/µL, indicating a large increase from a background fluorescence intensity. Therefore, it is understood that by employing the antibody fixed solid support of the present invention consisted of the solid support having the convex and concave properties comprising an organic substance, to which antibodies have been bound via sulfonyl group, a ligand having reactivity with the antibody fixed on the antibody fixed solid support can be efficiently detected.

### Example 11: Detection of ligand by antibody fixed slide

### (1) Fixation of antibody

Rabbit Anti Streptavidin (Polysienece) was diluted with PBS (100 ng/µL), and each 10 µL of it was pipetted into 384 wells plate. It was dotted on the solid support (A) prepared in the above-described Example 10 (1) using an arrayer made by Cartecyan (PixSys 5500). Immediately, the solid support after the dotting was left at 25°C for 1 hour in a saturated common salt chamber, then the blocking treatment was performed by immersing it in 1/4 x Block Ace (Dainippon Pharmaceuticals)/0.05% Tween 20-PBS (PBS-T) for 1 hour to obtain an antibody slide (C).

### (2) Labeling of ligand

1 mg of Streptavidin (Wako Junyaku) was dissolved in 400 *µ*l of 0.1 M NaHCO₃ (pH 8.0). It was transferred to a Cy3-monofunctional tube (Amersham Pharmacia Biotech) and incubated at room temperature for 30 minutes. 100 µl of 1 M Tris/HCl (pH 8.0) was added to it, and further incubated at room temperature for 30 minutes to stop the reaction. The reacted solution was purified by NAP-5 column (Amersham Pharmacia Biotech), and Cy3-labeled Streptavidin was obtained.

### (3) Reaction with ligand and detection of it

SecureSeal Hybridization Chamber (SA 500, Grace Bio-Labs) was intimately contacted to the antibody slide (C) prepared in the above-described (1) . Cy3-labeled Streptavidin obtained in the above-described (2) was diluted with 1/4 x Block Ace/PBS-T into 2 *µ*g/ml. After 500 *µ*L of it was added within SA 500, it was incubated at 25°C for 1 hour within a moisture chamber. Subsequently, it was washed with PBS-T three times, rinsed with PBS, and dried by a centrifuge treatment at 700 rpm for 5 minutes. When the fluorescence intensity of the slide glass surface was measured by a fluorescence scanning device, it was 26,300, indicating a large increase from the background fluorescence intensity. Therefore, it is understood that by employing the antibody fixed solid support of the present invention consisted of the solid support having the convex and concave properties comprising an organic substance to which antibodies have been bound via sulfonyl group, a ligand having reactivity with the antibody fixed on the antibody fixed solid support can be efficiently detected also in a microarray system.

### Industrial Applicability

By utilizing a fixation method of the present invention, a probe of a nucleotide derivative or its analog nucleotide such as an oligonucleotide, a polynucleotide, or a peptide nucleic acid can be fixed in high density with high stability on the surface of a solid support having the convex and concave portions. Therefore, the solid support to which the nucleotide derivative or its analog is fixed by the fixation method of the present invention forms the solid support with high stability, in which release of the probe resulted from hydrolysis hardly occurs. Particularly, in the case where an amino group or the like is introduced using a silane coupling agent to surface of the solid support, the probe can be firmly fixed to the solid support, since both of the binding of the amino group or the like to the surface of the solid support and the binding of the probe are formed by the covalent bond. A detection tool having a high detection limit which is capable of being utilized for gene analysis or the like can be obtained by this stable fixation of the probe.

As one example of it, a nucleic acid fragment sample having complementarity to the probe fixed on an oligonucleotide fixed solid support can be detected in a high sensitivity by performing the hybridization with the sample nucleic acid fragment using an oligonucleotide fixed solid support prepared according to the present invention. Moreover, a non-specific absorption of the sample nucleic acid fragment can be prevented by performing the treatment of the solid support surface with an anionic compound such as glycine after the probe sample such as the oligonucleotide is dotted on the surface of the reactive solid support, thereby exerting a larger effect on the detection with a high sensibility of a nucleic acid fragment sample having the complementarity.

Furthermore, according to the present invention, there can be provided a biological material chip wherein at least one member of specific binding partners is bound and fixed on a reactive solid support which is capable of rapidly and stably binding and fixing

## Claims

1. A reactive solid support having convex and concave portions on its surface, to which a group of vinyl sulfonyl groups or their reactive precursor groups are fixed by covalent bond via a linking group, respectively,
wherein the convex and concave portions comprise particles formed by an inorganic substance, or the convex and concave portions are formed by an organic substance, wherein the convex and concave portions are generated by coating the solid support with a particulate substance.

2. The reactive solid support as claimed in claim 1, wherein the convex and concave portions comprise a particle of silicon, alumina or titanium having the average diameter of 50 µm or less.

3. The reactive solid support as claimed in claim 1, wherein the organic substance is a macromolecular polymer or an aggregate of a macromolecular polymer.

4. The reactive solid support as claimed in claim 1, wherein a linked body of the vinylsulfonyl group or its reactive precursor group and the linking group is represented by the following formula:
-L-SO₂-X
in the above-described formula, X represents -CR¹=CR²R³ or -CHR^{I}-CR²R³Y, each of R¹, R² and R³ represents independently from each other an atom or a group selected from the group consisted of a hydrogen atom, an alkyl group having 1 to 6 carbon atoms, an aryl group having 6 to 20 carbon atoms, and an aralkyl group having 7 to 26 carbon atoms in total containing an alkyl chain having 1 to 6 carbon atoms; Y represents an atom or a group selected from the group consisted of a halogen atom, -OSO₂R¹¹, -OCOR¹², -OSO₃M and a quaternary pyridinium group; R¹¹ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; R¹² represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group.

5. The reactive solid support as claimed in claim 4, wherein X represents a vinyl group represented by -CH=CH₂.

6. The reactive solid support as claimed in claim 4, wherein L represents a linking group containing an atom of a bivalence or more except for carbon atom.

7. The reactive solid support as claimed in claim 4, wherein L represents a linking group having a linking portion selected from the group consisted of -NH-, -S- and -0-.

8. The reactive solid support as claimed in claim 4, wherein L represents a linking group represented by -(L¹)ₙ-NH-(CR¹R²)₂- or -(L¹)ₙ-S-(CR¹R²)₂- wherein R¹ and R² represents the same meanings as described above, L¹ represents a linking group, and n represents either 0 or 1.

9. The reactive solid support as claimed in claim 4, wherein L represents a linking group represented by -(L¹)ₙ-NHCH₂CH₂- wherein L¹ represents a linking group, and n represents either 0 or 1.

10. The reactive solid support as claimed in claim 8, wherein L¹ represents a linking group containing a group represented by -OSi-, and n represents 1.

11. The reactive solid support as claimed in claim 1, wherein said solid support is a substrate in a sheet shape selected from the group consisted of a glass substrate, a resin substrate, a glass substrate or a resin substrate surface-treated with a silane coupling agent and a glass substrate or a resin substrate having a covering layer on its surface.

12. The reactive solid support as claimed in claim 11, wherein said solid support is a substrate in a sheet shape selected from the group consisted of a silicate glass substrate, a silicate glass substrate surface-treated with a silane coupling agent and a silicate glass substrate covered by an organic covering layer.

13. A method of manufacturing the reactive solid support as claimed in claim 4, wherein a disulfone compound represented by the following formula is brought into contact with a reactive solid support to the surface of which a reactive group is introduced:
X¹-SO₂-L²-SO₂-X²
in the above-described formula, each of X¹ and X² represents independently from each other -CR¹=CR²R³ or -CHR¹-CR²R³Y, each of R¹, R² and R³ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, -OSO₂R¹¹, -OCOR¹², -OSO₃M and a quaternary pyridinium group; R¹¹ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; R¹² represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L² represents a linking group.

14. The method of manufacturing a reactive solid support as claimed in claim 13, in which the reactive group introduced to the surface of said solid support is an amino group, a mercapto group or a hydroxyl group.

15. A manufacturing method of a solid support comprising a nucleotide derivative or its analog bound to the surface of the support via a linking group having a sulfonyl group, wherein a surface of a reactive solid support having a convex and concave portion on which each of a group of vinylsulfonyl group or its reactive precursor group is fixed by covalent bond, is contacted with a nucleotide derivative or its analog having a reactive group which is capable of reacting with said reactive group to form a covalent bond, wherein the convex and concave portions are generated by coating the solid support with a particulate substance.

16. The manufacturing method as claimed in claim 15, wherein said nucleotide derivative or its analog is selected from the group consisted of an oligonucleotide, a polynucleotide and a peptide nucleic acid.

17. The manufacturing method as claimed in claim 15, wherein a reactive solid support that a linked body of a vinylsulfonyl group or its reactive precursor group represented by the following formula and a linking group is bound to and fixed on, is used as a reactive solid support having a convex and concave portion:
-L-SO₂-X
in the above-described formula, X represents -CR¹=CR²R³ or -CHR¹-CR²R³Y, each of R¹, R² and R³ represents independently from each other an atom or a group selected from the group consisted of a hydrogen atom, an alkyl group having 1 to 6 carbon atoms, an aryl group having 6 to 20 carbon atoms and an aralkyl group having 7 to 26 carbon atoms in total containing an alkyl chain having 1 to 6 carbon atoms; Y represents an atom or a group selected from the group consisted of a halogen atom, -OSO₂R¹¹, -OCOR¹² -OSO₃M and a quaternary pyridinium group; R¹¹ represents a group selected from the group consisted of an alkyl group having 1 to 6 carbon atoms, an aryl group having 6 to 20 carbon atoms and an aralkyl group having 7 to 26 carbon atoms in total containing an alkyl chain having 1 to 6 carbon atoms; R¹² represents a group selected from the group consisted of an alkyl group having 1 to 6 carbon atoms and a halogenated alkyl group having 1 to 6 carbon atoms; M represents an atom or a group selected from the group consisted of a hydrogen atom, an alkali metallic atom and an ammonium group; and L represents a linking group or a single bond.

18. The manufacturing method as claimed in claim 17, wherein X represents a reactive group represented by -CR¹=CR²R³ wherein each of R¹, R² and R³ represents the same meanings as described above.

19. A solid support having a convex and concave portion, in which a nucleotide derivative or its analog obtained by a manufacturing method claimed in any one of claims 15 to 18 is bound and fixed to surface of the solid support.

20. A method of binding and fixing a complementary oligonucleotide or polynucleotide, wherein the solid support having the convex and concave portion to which the nucleotide derivative or its analog is bound as claimed in claim 19 is contacted with an oligonucleotide or a polynucleotide having complementarity to said fixed nucleotide derivative or its analog in the presence of an aqueous medium.

21. The method as claimed in claim 20, wherein a detectable label is bound to said complementary oligonucleotide or polynucleotide.

22. A solid support to which a oligonucleotide or a polynucleotide having complementarity is bound and fixed wherein the solid support having the convex and concave portion to which, to the nucleotide derivative or its analog is bound as claimed in claim 19, a oligonucleotide or a polynucleotide having complementarity to said nucleotide derivative or its analog is bound in a complementary manner.

23. The solid support as claimed in claim 22, wherein a detectable label is bound to said oligonucleotide or polynucleotide having the complementarity.

24. A method of identifying or screening a gene, wherein the solid support having a convex and concave portion to the surface of which the nucleotide derivative or its analog is bound and fixed as claimed in claim 18, or the solid support to which the complementary oligonucleotide or polynucleotide is bound and fixed as claimed in claim 20 is utilized.

25. A biological material chip, wherein A, which represents a residue of at least one protein or protein binding substance, is bound to a solid support having a convex and concave portion by covalent bond via a sulfonyl group as the following formula (I):
Solid support-L-SO₂-X-A (I)
in the formula (I), L represents a linking group; X represents -CR¹ (R²) -R³ (R⁴)-; each of R¹, R², R³ and R⁴ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and A represents a residue of a protein or protein binding substance except for nucleic acid, wherein the convex and concave portions are generated by coating the solid support with a particulate substance.

26. The chip as claimed in claim 25, wherein said protein or protein binding substance bounded to the surface is an antibody, an antibody fragment, a ligand, an antigen, a hapten or a receptor.

27. The chip as claimed in claim 25, wherein said protein or protein binding substance bound to the surface is avidins.

28. The chip as claimed in claim 27, in which avidins are an avidin, a streptoavidin or altered bodies thereof which are capable of forming a stable complex with a biotin.

29. The chip as claimed in claim 25, wherein said protein bound to the surface is a nucleic acid recognition protein.

30. The chip as claimed in claim 29, in which said nucleic acid recognition protein is a double stranded DNA recognition protein.

31. The chip as claimed in claim 30, wherein said double stranded DNA recognition protein is a double stranded DNA recognition antibody.

32. The chip as claimed in claim 30, wherein said double stranded DNA recognition protein is a DNA transcription factor.

33. The chip as claimed in claim 30, wherein said double stranded DNA recognition protein is a protein having a Zinc finger motif or a Ring finger motif.

34. The chip as claimed in any one of claims 25 to 33, wherein said convex and concave portion comprises a particle formed by an inorganic substance.

35. The chip as claimed in claim 34, wherein said convex and concave portion comprises a particle containing silicon, alumina or titanium of which average particle diameter is 50 *µ*m or less.

36. The chip as claimed in any of claims 25 to 33,in which said convex and concave portion is formed by an organic substance.

37. The chip as claimed in claim 36, wherein said organic substance comprises a high molecular polymer or an aggregate of a high molecular polymer.

38. The chip as claimed in claim 25, wherein said solid support is a glass, a plastic, an electrode surface or a sensor chip surface.

39. A method of detecting a target substance, comprising the steps of:
contacting the chip claimed in claim 25 with a sample containing a target substance which specifically binds to said protein or protein binding substance except for nucleic acid supported on the surface of said chip; and
detecting formation of reciprocal binding between said protein or protein binding substance and said target substance.

40. The method of detecting a target substance as claimed in claim 39 wherein said target substance is labeled with at least one component capable of generating a detectable signal.

41. The method of detecting a target substance as claimed in claim 39, comprising a step of performing blockingprocessing of the chip with an aqueous solution of an amino acid, a peptide or a protein.

42. A method of manufacturing the chip claimed in claim 25 comprising a step in which a solid support having a convex and concave portion containing a vinylsulfonyl group or its reactive precursor group represented by the following formula (II) on its surface is contacted with at least one protein or protein binding substance having a reactive group which forms a covalent bond by reacting with said vinylsulfonyl group or its reactive precursor group:
-L-SO₂-X' (II)
in the above-described formula (II), L represents a linking group which binds -L-SO₂-X' to a solid support; X' represents -CR¹=CR² (R³) or -CH (R¹) -CR² (R³) (Y); each of R¹, R² and R³ represents independently from each other a hydrogen atom, an alkyl group having 1 to 6 carbon atoms, an aryl group having 6 to 20 carbon atoms or an aralkyl group having 7 to 26 carbon atoms in total containing an alkyl chain having 1 to 6 carbon atoms; and Y represents a group which is substituted by a neucleophilic reagent or a group which is eliminated as a "HY" by a base.

43. The method of manufacturing a chip as claimed in claim 42, wherein said protein or protein binding substance bound to the surface is an antibody, an antibody fragment, a ligand, an antigen, a hapten or a receptor.

44. The method of manufacturing a chip as claimed in claim 42, wherein said protein or protein binding substance bound to the surface is avidins.

45. The method of manufacturing a chip as claimed in claim 44, wherein avidins are an avidin, a streptoavidin or altered bodies thereof which are capable of forming a stable complex with a biotin.

46. The method of manufacturing a chip as claimed in claim 42, wherein said protein bound to the surface is a nucleic acid recognition protein.

47. The method of manufacturing a chip as claimed in claim 46, wherein said nucleic acid recognition protein is a double stranded DNA recognition protein.

48. The method of manufacturing a chip as claimed in claim 47, wherein said double stranded DNA recognition protein is a double stranded DNA recognition antibody.

49. The method of manufacturing a chip as claimed in claim 47, wherein said double stranded DNA recognition protein is a DNA transcription factor.

50. The method of manufacturing a chip as claimed in claim 47, wherein said double stranded DNA recognition protein is a protein having a Zinc finger motif or a Ring finger motif.

51. The method of manufacturing a chip as claimed in claim 42, wherein said convex and concave portion comprises a particle formed by an inorganic substance.

52. The method of manufacturing a chip as claimed in claim 34, wherein said convex and concave portion comprises a particle containing silicon, alumina or titanium of which average particle diameter is 50 µm or less.

53. The method of manufacturing a chip as claimed in claim 42, in which said convex and concave portion is formed by an organic substance.

54. The method of manufacturing a chip as claimed in claim 53, wherein said organic substance is a high molecular polymer or an aggregate of a high molecular polymer.

55. The method of manufacturing a chip as claimed in claim 42, in which a solid support is a glass, a plastic, an electrode surface or a sensor chip surface.

56. The method of manufacturing a chip as claimed in claim 42, comprising the steps of:
fixing at least one protein or protein binding substance to a solid support having convex and concave portions by contacting said protein or protein binding substance to said solid support; and
performing blocking process of a free vinylsulfonyl group or its reactive precursor group located on the surface of said solid support with an amino acid, a peptide or a protein aqueous solution.

## Patentansprüche

1. Reaktiver fester Träger, der konvexe und konkave Anteile auf seiner Oberfläche aufweist, an welcher eine Gruppe von Vinylsulfonylgruppen bzw. ihre reaktiven Vorläufergruppen durch kovalente Bindung über eine Verknüpfungsgruppe fixiert sind,
wobei die konvexen und konkaven Anteile Partikel, gebildet von einer anorganischen Substanz, umfassen oder die konvexen und konkaven Anteile von einer organischen Substanz gebildet werden, wobei die konvexen und konkaven Anteile durch Überziehen des festen Trägers mit einer partikulären Substanz erzeugt sind.

2. Reaktiver fester Träger nach Anspruch 1, wobei die konvexen und konkaven Anteile ein Partikel aus Silicium, Aluminiumoxid oder Titan, das den durchschnittlichen Durchmesser von 50 µm oder weniger aufweist, umfassen.

3. Reaktiver fester Träger nach Anspruch 1, wobei die organische Substanz ein makromolekulares Polymer oder ein Aggregat eines makromolekularen Polymers ist.

4. Reaktiver fester Träger nach Anspruch 1, wobei ein verknüpfter Körper der Vinylsulfonylgruppe oder ihrer reaktiven Vorläufergruppe und der Verknüpfungsgruppe durch die folgende Formel dargestellt ist:
-L-SO₂-X
in der oben beschriebenen Formel stellt X -CR¹=CR²R³ oder -CHR¹-CR²R³Y dar, stellt jeder von R¹, R² und R³ unabhängig voneinander ein Atom oder eine Gruppe, ausgewählt aus der Gruppe, bestehend aus einem Wasserstoffatom, einer Alkylgruppe mit 1 bis 6 Kohlenstoffatomen, einer Arylgruppe mit 6 bis 20 Kohlenstoffatomen und einer Aralkylgruppe mit 7 bis 26 Kohlenstoffatomen insgesamt, enthaltend eine Alkylkette mit 1 bis 6 Kohlenstoffatomen, dar; stellt Y ein Atom oder eine Gruppe, ausgewählt aus der Gruppe, bestehend aus einem Halogenatom, -OSO₂R¹¹, -OCOR¹², -OSO₃M und einer quaternären Pyridiniumgruppe, dar; stellt R¹¹ eine Gruppe dar, ausgewählt aus der Gruppe, bestehend aus einer Alkylgruppe mit 1 bis 6 Kohlenstoffatomen, einer Arylgruppe mit 6 bis 20 Kohlenstoffatomen und einer Aralkylgruppe mit 7 bis 26 Kohlenstoffatomen insgesamt, enthaltend eine Alkylkette mit 1 bis 6 Kohlenstoffatomen; stellt R¹² eine Gruppe dar, ausgewählt aus der Gruppe, bestehend aus einer Alkylgruppe mit 1 bis 6 Kohlenstoffatomen und einer halogenierten Alkylgruppe mit 1 bis 6 Kohlenstoffatomen; stellt M ein Atom oder eine Gruppe, ausgewählt aus der Gruppe, bestehend aus einem Wasserstoffatom, einem Alkalimetallatom und einer Ammoniumgruppe, dar; und stellt L eine Verknüpfungsgruppe dar.

5. Reaktiver fester Träger nach Anspruch 4, wobei X eine Vinylgruppe, dargestellt durch -CH=CH₂, darstellt.

6. Reaktiver fester Träger nach Anspruch 4, wobei L eine Verknüpfungsgruppe, enthaltend ein Atom von einer Hivalenz oder mehr, mit Ausnahme eines Kohlenstoffatoms, darstellt.

7. Reaktiver fester Träger nach Anspruch 4, wobei L eine Verknüpfungsgruppe mit einem Verknüpfungsanteil, ausgewählt aus der Gruppe, bestehend aus -NH-, -S- und -O-, darstellt.

8. Reaktiver fester Träger nach Anspruch 4, wobei L eine Verknüpfungsgruppe, dargestellt durch -(L¹)ₙ-NH-(CR¹CR²)₂- oder -(L¹)ₙ-S-(CR¹R²)₂-, wobei R¹ und R² die gleichen Bedeutungen repräsentieren wie oben beschrieben, L¹ eine Verknüpfungsgruppe darstellt und n entweder 0 oder 1 darstellt, darstellt.

9. Reaktiver fester Träger nach Anspruch 4, wobei L eine Verknüpfungsgruppe, dargestellt durch -(L¹)ₙ-NHCH₂CH₂-, wobei L¹ eine Verknüpfungsgruppe darstellt und n entweder 0 oder 1 darstellt, darstellt.

10. Reaktiver fester Träger nach Anspruch 8, wobei L¹ eine Verknüpfungsgruppe, enthaltend eine Gruppe, dargestellt durch -OSi-, darstellt, und n 1 darstellt.

11. Reaktiver fester Träger nach Anspruch 1, wobei der feste Träger ein Substrat in einer Blattform, ausgewählt aus der Gruppe, bestehend aus einem Glassubstrat, einem Harzsubstrat, einem Glassubstrat oder einem Harzsubstrat, oberflächenbehandelt mit einem Silan-Haftvermittler, und einem Glassubstrat oder einem Harzsubstrat, das eine bedeckende Schicht auf seiner Oberfläche aufweist, ist.

12. Reaktiver fester Träger nach Anspruch 11, wobei der feste Träger ein Substrat in einer Blattform, ausgewählt aus der Gruppe, bestehend aus einem Silicatglassubstrat, einem Silicatglassubstrat, oberflächenbehandelt mit einem Silan-Haftvermittler, und einem Silicatglassubstrat, bedeckt mit einer organischen Deckschicht, ist.

13. Verfahren zur Herstellung des reaktiven festen Trägers nach Anspruch 4, wobei eine Disulfonverbindung, dargestellt durch die folgende Formel, in Kontakt gebracht wird mit einem reaktiven festen Träger, an dessen Oberfläche eine reaktive Gruppe eingeführt ist:
X¹-SO₂-L²-SO₂-X²
in der oben beschriebenen Formel stellen jeder von X¹ und X² unabhängig voneinander -CR¹=CR²R³oder -CHR¹-CR²R³Y dar, stellt jeder von R¹, R² und R³ unabhängig voneinander ein Atom oder eine Gruppe, ausgewählt aus der Gruppe, bestehend aus einem Wasserstoffatom, einer Alkylgruppe mit 1 bis 6 Kohlenstoffatomen, einer Arylgruppe mit 6 bis 20 Kohlenstoffatomen und einer Aralkylgruppe mit 7 bis 26 Kohlenstoffatomen insgesamt, enthaltend eine Alkylkette mit 1 bis 6 Kohlenstoffatomen, dar; stellt Y ein Atom oder eine Gruppe, ausgewählt aus der Gruppe, bestehend aus einem Halogenatom, -OSO₂R¹¹, -OCOR¹², -OSO₃M und einer quaternären Pyridiniumgruppe, dar; stellt R¹¹ eine Gruppe dar, ausgewählt aus der Gruppe, bestehend aus einer Alkylgruppe mit 1 bis 6 Kohlenstoffatomen, einer Arylgruppe mit 6 bis 20 Kohlenstoffatomen und einer Aralkylgruppe mit 7 bis 26 Kohlenstoffatomen insgesamt, enthaltend eine Alkylkette mit 1 bis 6 Kohlenstoffatomen; stellt R¹² eine Gruppe dar, ausgewählt aus der Gruppe, bestehend aus einer Alkylgruppe mit 1 bis 6 Kohlenstoffatomen und einer halogenierten Alkylgruppe mit 1 bis 6 Kohlenstoffatomen; stellt M ein Atom oder eine Gruppe, ausgewählt aus der Gruppe, bestehend aus einem Wasserstoffatom, einem Alkalimetallatom und einer Ammoniumgruppe, dar; und stellt L² eine Verknüpfungsgruppe dar.

14. Verfahren zur Herstellung eines reaktiven festen Trägers nach Anspruch 13, in welchem die reaktive Gruppe, eingeführt an die Oberfläche des festen Trägers, eine Aminogruppe, eine Mercaptogruppe oder eine Hydroxylgruppe ist.

15. Herstellungsverfahren eines festen Trägers, umfassend ein Nucleotidderivat oder sein Analogon, gebunden an die Oberfläche des Trägers über eine Verknüpfungsgruppe, die eine Sulfonylgruppe aufweist, wobei eine Oberfläche eines reaktiven festen Trägers mit einem konvexen und konkaven Anteil, auf welcher jeder einer Gruppe von Vinylsulfonylgruppe oder ihrer reaktiven Vorläufergruppe durch kovalente Bindung fixiert ist, in Kontakt gebracht wird mit einem Nucleotidderivat oder seinem Analogon mit einer reaktiven Gruppe, welches in der Lage ist, mit der reaktiven Gruppe zu reagieren, um eine kovalente Bindung zu bilden, wobei die konvexen und konkaven Anteile durch Überziehen des festen Trägers mit einer partikulären Substanz erzeugt sind.

16. Herstellungsverfahren nach Anspruch 15, wobei das Nucleotidderivat oder sein Analogon ausgewählt ist aus der Gruppe, bestehend aus einem Oligonucleotid, einem Polynucleotid und einer Peptidnucleinsäure.

17. Herstellungsverfahren nach Anspruch 15, wobei ein reaktiver fester Träger, an den ein verknüpfter Körper aus einer Vinylsulfonylgruppe oder ihrer reaktiven Vorläufergruppe, dargestellt durch die folgende Formel, und einer Verknüpfungsgruppe, gebunden ist und daran fixiert ist, verwendet wird als ein reaktiver fester Träger mit einem konvexen und konkaven Anteil:
-L-SO₂-X
in der oben beschriebenen Formel stellt X -CR¹=CR²R³ oder -CHR¹-CR²R³Y dar, stellt jeder von R¹, R² und R³ unabhängig voneinander ein Atom oder eine Gruppe, ausgewählt aus der Gruppe bestehend aus einem Wasserstoffatom, einer Alkylgruppe mit 1 bis 6 Kohlenstoffatomen, einer Arylgruppe mit 6 bis 20 Kohlenstoffatomen und einer Aralkylgruppe mit 7 bis 26 Kohlenstoffatomen insgesamt, enthaltend eine Alkylkette mit 1 bis 6 Kohlenstoffatomen, dar; stellt Y ein Atom oder eine Gruppe, ausgewählt aus der Gruppe; bestehend aus einem Halogenatom, -OSO₂R¹¹, -OCOR¹², -OSO₃M und einer quaternären Pyridiniumgruppe, dar; stellt R¹¹ eine Gruppe dar, ausgewählt aus der Gruppe, bestehend aus einer Alkylgruppe mit 1 bis 6 Kohlenstoffatomen, einer Arylgruppe mit 6 bis 20 Kohlenstoffatomen und einer Aralkylgruppe mit 7 bis 26 Kohlenstoffatomen insgesamt, enthaltend eine Alkylkette mit 1 bis 6 Kohlenstoffatomen; stellt R¹² eine Gruppe dar, ausgewählt aus der Gruppe, bestehend aus einer Alkylgruppe mit 1 bis 6 Kohlenstoffatomen und einer halogenierten Alkylgruppe mit 1 bis 6 Kohlenstoffatomen; stellt M ein Atom oder eine Gruppe, ausgewählt aus der Gruppe, bestehend aus einem Wasserstoffatom, einem Alkalimetallatom und einer Ammoniumgruppe, dar; und stellt L eine Verknüpfungsgruppe oder eine Einfachbindung dar.

18. Herstellungsverfahren nach Anspruch 17, wobei X eine reaktive Gruppe, dargestellt durch -CR¹=CR²R³, wobei jeder von R¹, R² und R³ die gleichen Bedeutungen wie oben beschrieben darstellt, darstellt.

19. Fester Träger mit einem konvexen und konkaven Anteil, in welchem ein Nucleotidderivat oder sein Analogon, erhalten durch ein Herstellungsverfahren nach einem der Ansprüche 15 bis 18, gebunden und fixiert ist an die Oberfläche des festen Trägers.

20. Verfahren, ein komplementäres Oligonucleotid oder Polynucleotid zu binden und zu fixieren, wobei der feste Träger mit dem konvexen und konkaven Anteil, an welchen das Nucleotidderivat oder sein Analogon gebunden ist, nach Anspruch 19, in Gegenwart eines wässrigen Mediums mit einem Oligonucleotid oder einem Polynucleotid in Kontakt gebracht wird, das Komplementarität zu dem fixierten Nucleotidderivat oder seinem Analogon aufweist.

21. Verfahren nach Anspruch 20, wobei eine detektierbare Markierung an das komplementäre Oligonucleotid oder Polynucleotid gebunden ist.

22. Fester Träger nach Anspruch 19, an welchen/m ein Oligonucleotid oder ein Polynucleotid, das Komplementarität aufweist, gebunden und fixiert ist, wobei der feste Träger mit dem konvexen und konkaven Anteil an das daran gebundene Nucleotidderivat oder sein Analogon ein Oligonucleotid oder ein Polynucleotid, das Komplementarität zu dem Nucleotidderivat oder seinem Analogon aufweist, auf eine komplementäre Weise gebunden hat.

23. Fester Träger nach Anspruch 22, wobei eine detektierbare Markierung gebunden ist an das Oligonucleotid oder Polynucleotid, das die Komplementarität aufweist.

24. Verfahren zum Identifizieren oder Screenen eines Gens, wobei der feste Träger mit einem konvexen und konkaven Anteil, an dessen Oberfläche das Nucleotidderivat oder sein Analogon gebunden und fixiert ist, nach Anspruch 18, oder der feste Träger, an welchem/n das komplementäre Oligonucleotid oder Polynucleotid gebunden und fixiert ist, nach Anspruch 20, verwendet wird.

25. Biologisches Material-Chip, wobei A, welches einen Rest von mindestens einem Protein oder einer Protein-bindenden Substanz darstellt, an einen festen Träger mit einem konvexen und konkaven Anteil über eine Sulfonylgruppe, wie die folgende Formel (I), durch kovalente Bindung gebunden ist:
Fester Träger-L-SO₂-X-A (I)
in der Formel (I) stellt L eine Verknüpfungsgruppe dar; stellt X -CR¹(R²)-R³(R⁴)- dar; stellt jeder von R¹, R², R³ und R⁴ unabhängig voneinander ein Wasserstoffatom, eine Alkylgruppe mit 1 bis 6 Kohlenstoffatomen, eine Arylgruppe mit 6 bis 20 Kohlenstoffatomen oder eine Aralkylgruppe mit 7 bis 26 Kohlenstoffatomen insgesamt, enthaltend eine Alkylkette mit 1 bis 6 Kohlenstoffatomen, dar; und stellt A einen Rest eines Proteins oder einer Protein-bindenden Substanz, außer einer Nucleinsäure, dar, wobei die konvexen und konkaven Anteile durch Beschichten des festen Trägers mit einer partikulären Substanz erzeugt werden.

26. Chip nach Anspruch 25, wobei das Protein oder die Protein-bindende Substanz, gebunden an die Oberfläche, ein Antikörper, ein Antikörperfragment, ein Ligand, ein Antigen, ein Hapten oder ein Rezeptor ist.

27. Chip nach Anspruch 25, wobei das Protein oder die Protein-bindende Substanz, gebunden an die Oberfläche, Avidine ist.

28. Chip nach Anspruch 27, in welchem Avidine ein Avidin, ein Streptavidin oder veränderte Körper davon sind, welche fähig sind, einen stabilen Komplex mit einem Biotin zu bilden.

29. Chip nach Anspruch 25, wobei das an die Oberfläche gebundene Protein ein Nucleinsäure-Erkennungsprotein ist.

30. Chip nach Anspruch 29, in welchem das Nucleinsäure-Erkennungsprotein ein doppelsträngige DNA-Erkennungsprotein ist.

31. Chip nach Anspruch 30, wobei das doppelsträngige DNA-Erkennungsprotein ein doppelsträngige DNA-Erkennungsantikörper ist.

32. Chip nach Anspruch 30, wobei das doppelsträngige DNA-Erkennungsprotein ein DNA-Transkriptionsfaktor ist.

33. Chip nach Anspruch 30, wobei das doppelsträngige DNA-Erkennungsprotein ein Protein ist, das ein Zinkfingermotiv oder ein Ringfingermotiv aufweist.

34. Chip nach einem der Ansprüche 25 bis 33, wobei der konvexe und konkave Anteil ein Partikel umfasst, das von einer anorganischen Substanz gebildet wird.

35. Chip nach Anspruch 34, wobei der konvexe und konkave Anteil ein Partikel, enthaltend Silicium, Aluminiumoxid oder Titan, von welchem der durchschnittliche Partikeldurchmesser 50 µm oder weniger ist, umfasst.

36. Chip nach einem der Ansprüche 25 bis 33, in welchem der konvexe und konkave Anteil von einer organischen Substanz gebildet wird.

37. Chip nach Anspruch 36, wobei die organische Substanz ein hochmolekulares Polymer oder ein Aggregat eines hochmolekularen Polymers umfasst.

38. Chip nach Anspruch 25, wobei der feste Träger ein Glas, ein Kunststoff, eine Elektrodenoberfläche oder eine Sensorchipoberfläche ist.

39. Verfahren zum Detektieren einer Zielsubstanz, umfassend die folgenden Schritte:
In-Kontakt-Bringen des Chips nach Anspruch 25 mit einer Probe, enthaltend eine Zielsubstanz, welche spezifisch an das Protein oder die Protein-bindende Substanz außer Nucleinsäure, getragen auf der Oberfläche des Chips, bindet; und
Detektieren der Ausbildung reziproken Bindens zwischen dem Protein oder der Protein-bindenden Substanz und der Zielsubstanz.

40. Verfahren zum Detektieren einer Zielsubstanz nach Anspruch 39, wobei die Zielsubstanz mit wenigstens einer Komponente, fähig zum Erzeugen eines detektierbaren Signals, markiert ist.

41. Verfahren zum Detektieren einer Zielsubstanz nach Anspruch 39, umfassend einen Schritt des Durchführens einer Blockierungsprozessierung des Chips mit einer wässrigen Lösung einer Aminosäure, eines Peptids oder eines Proteins.

42. Verfahren zum Herstellen des Chips nach Anspruch 25, umfassend einen Schritt, in welchem ein fester Träger mit einem konvexen und konkaven Anteil, enthaltend eine Vinylsulfonylgruppe oder deren reaktive Vorläufergruppe, dargestellt durch die folgende Formel (II), auf seiner Oberfläche, mit wenigstens einem Protein oder einer Protein-bindenden Substanz mit einer reaktiven Gruppe, welche eine kovalente Bindung durch Reagieren mit der Vinylsulfonylgruppe oder ihrer reaktiven Vorläufergruppe ausbildet, in Kontakt gebracht wird:
-L-SO₂-X' (II)
in der oben beschriebenen Formel (II) stellt L eine Verknüpfungsgruppe dar, welche -L-SO₂-X' an einen festen Träger bindet; stellt X' -CR¹=CR² (R³) oder -CH(R¹)-CR²(R³) (Y) dar; stellt jeder von R¹, R² und R³ unabhängig voneinander ein Wasserstoffatom, eine Alkylgruppe mit 1 bis 6 Kohlenstoffatomen, eine Arylgruppe mit 6 bis 20 Kohlenstoffatomen oder eine Aralkylgruppe mit 7 bis 26 Kohlenstoffatomen insgesamt, enthaltend eine Alkylkette mit 1 bis 6 Kohlenstoffatomen, dar; und stellt Y eine Gruppe dar, welche substituiert ist mit einem nucleophilen Reagenz ("neucleophilic reagent") oder eine Gruppe dar, welche als ein "HY" mittels einer Base eliminiert wird.

43. Verfahren zur Herstellung eines Chips nach Anspruch 42, wobei das Protein oder die Protein-bindende Substanz, gebunden an die Oberfläche, ein Antikörper, ein Antikörperfragment, ein Ligand, ein Antigen, ein Hapten oder ein Rezeptor ist.

44. Verfahren zur Herstellung eines Chips nach Anspruch 42, wobei das Protein oder die Protein-bindende Substanz, gebunden an die Oberfläche, Avidine ist.

45. Verfahren zur Herstellung eines Chips nach Anspruch 44, wobei Avidine ein Avidin, ein Streptavidin oder veränderte Körper davon sind, welche in der Lage sind, einen stabilen Komplex mit einem Biotin auszubilden.

46. Verfahren zur Herstellung eines Chips nach Anspruch 42, wobei das Protein, gebunden an die Oberfläche, ein Nucleinsäure-Erkennungsprotein ist.

47. Verfahren zur Herstellung eines Chips nach Anspruch 46, wobei das Nucleinsäure-Erkennungsprotein ein doppelsträngige DNA-Erkennungsprotein ist.

48. Verfahren zur Herstellung eines Chips nach Anspruch 47, wobei das doppelsträngige DNA-Erkennungsprotein ein dopppelsträngige DNA-Erkennungsantikörper ist.

49. Verfahren zur Herstellung eines Chips nach Anspruch 47, wobei das doppelsträngige DNA-Erkennungsprotein ein DNA-Transkriptionsfaktor ist.

50. Verfahren zur Herstellung eines Chips nach Anspruch 47, wobei das doppelsträngige DNA-Erkennungsprotein ein Protein ist, das ein Zinkfingermotiv oder ein Ringfingermotiv aufweist.

51. Verfahren zur Herstellung eines Chips nach Anspruch 42, wobei der konvexe und konkave Anteil ein Partikel, gebildet von einer anorganischen Substanz, umfasst.

52. Verfahren zur Herstellung eines Chips nach Anspruch 34, wobei der konvexe und konkave Anteil ein Partikel, enthaltend Silicium, Aluminiumoxid oder Titan, von welchem der durchschnittliche Partikeldurchmesser 50 µm oder weniger ist, umfasst.

53. Verfahren zur Herstellung eines Chips nach Anspruch 42, in welchem der konvexe und konkave Anteil von einer organischen Substanz gebildet wird.

54. Verfahren zur Herstellung eines Chips nach Anspruch 53, wobei die organische Substanz ein hochmolekulares Polymer oder ein Aggregat eines hochmolekularen Polymers ist.

55. Verfahren zur Herstellung eines Chips nach Anspruch 42, in welchem ein fester Träger ein Glas, ein Kunststoff, eine Elektrodenoberfläche oder eine Sensorchip-Oberfläche ist.

56. Verfahren zur Herstellung eines Chips nach Anspruch 42, umfassend die folgenden Schritte:
Fixieren wenigstens eines Proteins oder einer Protein-bindenden Substanz an einen festen Träger mit konvexen und konkaven Anteilen durch In-Kontakt-Bringen des Proteins oder der Protein-bindenden Substanz mit dem festen Träger; und
Durchführen eines Blockierungsprozesses für eine freie Vinylsulfonylgruppe oder ihre reaktive Vorläufergruppe, lokalisiert auf der Oberfläche des festen Trägers, mit einer wässrigen Aminosäure-, Peptid- oder Proteinlösung.

## Revendications

1. Support solide réactif ayant des parties convexes et concaves sur sa surface, sur lequel un groupe de groupements vinyl-sulfonyle ou leurs groupes précurseurs réactifs sont fixés par liaison covalente par l'intermédiaire d'un groupe de liaison, respectivement,
dans lequel les parties convexes et concaves comprennent des particules formées par une substance inorganique, ou les parties convexes et concaves sont formées par une substance organique, dans lequel les parties convexes et concaves sont générées par revêtement du support solide avec une substance particulaire.

2. Support solide réactif tel que défini dans la revendication 1, dans lequel les parties convexes et concaves comprennent une particule de silicium, d'alumine ou de titane ayant le diamètre moyen de 50 µm ou moins.

3. Support solide réactif tel que défini dans la revendication 1, dans lequel la substance organique est un polymère macromoléculaire ou un agrégat d'un polymère macromoléculaire.

4. Support solide réactif tel que défini dans la revendication 1, dans lequel un corps lié du groupement vinyl-sulfonyle ou son groupe précurseur réactif et du groupe de liaison est représenté par la formule suivante :
-L-SO₂-X
dans la formule décrite ci-dessus, X représente -CR ¹=CR²R³ ou -CHR¹-CR²R³Y, chacun parmi R¹, R²et R³ représente indépendamment l'un de l'autre un atome ou un groupe choisi dans le groupe consistant en un atome d'hydrogène, un groupe alkyle ayant 1 à 6 atomes de carbone, un groupe aryle ayant 6 à 20 atomes de carbone, et un groupe aralkyle ayant 7 à 26 atomes de carbone au total contenant une chaîne alkyle ayant 1 à 6 atomes de carbone ; Y représente un atome ou un groupe choisi dans le groupe consistant en un atome d'halogène, -OSO₂R¹¹, -OCOR¹², -OSO₃M et un groupe pyridine quaternaire ; R¹¹ représente un groupe choisi dans le groupe consistant en un groupe alkyle ayant 1 à 6 atomes de carbone, un groupe aryle ayant 6 à 20 atomes de carbone et un groupe aralkyle ayant 7 à 26 atomes de carbone au total contenant une chaîne alkyle ayant 1 à 6 atomes de carbone ; R¹² représente un groupe choisi dans le groupe consistant en un groupe alkyle ayant 1 à 6 atomes de carbone et un groupe alkyle halogéné ayant 1 à 6 atomes de carbone ; M représente un atome ou un groupe choisi dans le groupe consistant en un atome d'hydrogène, un atome métallique alcalin et un groupe ammonium ; et L représente un groupe de liaison.

5. Support solide réactif tel que défini dans la revendication 4, dans lequel X représente un groupe vinyle représenté par -CH=CH₂.

6. Support solide réactif tel que défini dans la revendication 4, dans lequel L représente un groupe de liaison contenant un atome d'une bivalence ou plus à l'exception de l'atome de carbone.

7. Support solide réactif tel que défini dans la revendication 4, dans lequel L représente un groupe de liaison ayant un segment de liaison choisi dans le groupe consistant en -NH-, -S- et -O-.

8. Support solide réactif tel que défini dans la revendication 4, dans lequel L représente un groupe de liaison représenté par -(L¹)₀-NH-(CR¹R²)₂- ou -(L¹)₀-S-(CR¹R²)₂- dans lequel R¹ et R² représentent les mêmes significations que celles décrites ci-dessus, L¹ représente un groupe de liaison, et n représente soit 0 soit 1.

9. Support solide réactif tel que défini dans la revendication 4, dans lequel L représente un groupe de liaison représenté par -(L¹)₀-NHCH₂CH₂- dans lequel L¹ représente un groupe de liaison, et n représente soit 0 soit 1.

10. Support solide réactif tel que défini dans la revendication 8, dans lequel L¹ représente un groupe de liaison contenant un groupe représenté par -OSi-, et n représente 1.

11. Support solide réactif tel que défini dans la revendication 1, dans lequel ledit support solide est un substrat en forme de feuille choisi dans le groupe consistant en un substrat en verre, un substrat en résine, une surface d'un substrat en verre ou d'un substrat en résine traitée avec un agent de pontage à base de silane et un substrat en verre ou un substrat en résine ayant une couche de revêtement sur sa surface.

12. Support solide réactif tel que défini dans la revendication 11, dans lequel ledit support solide est un substrat en forme de feuille choisi dans le groupe consistant en un substrat en verre à base de silicate, une surface d'un substrat en verre à base de silicate traitée avec un agent de pontage à base de silane et un substrat en verre à base de silicate revêtu avec une couche de revêtement organique.

13. Procédé de fabrication du support solide réactif tel que défini dans la revendication 4, dans lequel un composé disulfone représenté par la formule suivante est mis en contact avec un support solide réactif sur la surface duquel un groupe réactif est introduit :
X¹-SO₂-L²-SO₂-X²
dans la formule décrite ci-dessus, chacun parmi X¹ et X² représente indépendamment l'un de l'autre -CR¹=CR²R³ ou -CHR¹-CR²R³Y, chacun parmi R¹, R² et R³ représente indépendamment l'un de l'autre un atome ou un groupe choisi dans le groupe consistant en un atome d'hydrogène, un groupe alkyle ayant 1 à 6 atomes de carbone, un groupe aryle ayant 6 à 20 atomes de carbone et un groupe aralkyle ayant 7 à 26 atomes de carbone au total contenant une chaîne alkyle ayant 1 à 6 atomes de carbone ; Y représente un atome ou un groupe choisi dans le groupe consistant en un atome d'halogène, -OSO₂R¹¹, -OCOR¹², -OSO₃M et un groupe pyridine quaternaire ; R¹¹ représente un groupe choisi dans le groupe consistant en un groupe alkyle ayant 1 à 6 atomes de carbone, un groupe aryle ayant 6 à 20 atomes de carbone et un groupe aralkyle ayant 7 à 26 atomes de carbone au total contenant une chaîne alkyle ayant 1 à 6 atomes de carbone ; R¹² représente un groupe choisi dans le groupe consistant en un groupe alkyle ayant 1 à 6 atomes de carbone et un groupe alkyle halogéné ayant 1 à 6 atomes de carbone ; M représente un atome ou un groupe choisi dans le groupe consistant en un atome d'hydrogène, un atome métallique alcalin et un groupe ammonium ; et L² représente un groupe de liaison.

14. Procédé de fabrication d'un support solide réactif tel que défini dans la revendication 13, dans lequel le groupe réactif introduit sur la surface dudit support solide est un groupe amino, un groupe mercapto ou un groupe hydroxyle.

15. Procédé de fabrication d'un support solide comprenant un dérivé nucléotidique ou son analogue lié à la surface du support par l'intermédiaire d'un groupe de liaison ayant un groupe sulfonyle, dans lequel une surface d'un support solide réactif ayant une partie convexe et concave sur laquelle chacun parmi un groupe de groupement vinyl-sulfonyle ou son groupe précurseur réactif est fixé par liaison covalente, est mise en contact avec un dérivé nucléotidique ou son analogue ayant un groupe réactif qui est capable de réagir avec ledit groupe réactif pour former une liaison covalente, dans lequel les parties convexes et concaves sont générées par revêtement du support solide avec une substance particulaire.

16. Procédé de fabrication tel que défini dans la revendication 15, dans lequel ledit dérivé nucléotidique ou son analogue est choisi dans le groupe consistant en un oligonucléotide, un polynucléotide et un acide nucléique peptidique.

17. Procédé de fabrication tel que défini dans la revendication 15, dans lequel un support solide réactif auquel est lié et sur lequel est fixé un corps lié d'un groupement vinyl-sulfonyle ou son groupe précurseur réactif représenté par la formule suivante et d'un groupe de liaison, est utilisé en tant que support solide réactif ayant une partie convexe et concave :
-L-SO₂-X
dans la formule décrite ci-dessus, X représente -CR¹=CR²R³ ou -CHR¹-CR²R³Y, chacun parmi R¹, R² et R³ représente indépendamment l'un de l'autre un atome ou un groupe choisi dans le groupe consistant en un atome d'hydrogène, un groupe alkyle ayant 1 à 6 atomes de carbone, un groupe aryle ayant 6 à 20 atomes de carbone et un groupe aralkyle ayant 7 à 26 atomes de carbone au total contenant une chaîne alkyle ayant 1 à 6 atomes de carbone ; Y représente un atome ou un groupe choisi dans le groupe consistant en un atome d'halogène, -OSO₂R¹¹, -OCOR¹², -OSO₃M et un groupe pyridine quaternaire ; R¹¹ représente un groupe choisi dans le groupe consistant en un groupe alkyle ayant 1 à 6 atomes de carbone, un groupe aryle ayant 6 à 20 atomes de carbone et un groupe aralkyle ayant 7 à 26 atomes de carbone au total contenant une chaîne alkyle ayant 1 à 6 atomes de carbone ; R¹² représente un groupe choisi dans le groupe consistant en un groupe alkyle ayant 1 à 6 atomes de carbone et un groupe alkyle halogéné ayant 1 à 6 atomes de carbone ; M représente un atome ou un groupe choisi dans le groupe consistant en un atome d'hydrogène, un atome métallique alcalin et un groupe ammonium ; et L représente un groupe de liaison ou une liaison simple.

18. Procédé de fabrication tel que défini dans la revendication 17, dans lequel X représente un groupe réactif représenté par -CR¹=CR²R³ dans lequel chacun parmi R¹, R² et R³ représente les mêmes significations que celles décrites ci-dessus.

19. Support solide ayant une partie convexe et concave, dans lequel un dérivé nucléotidique ou son analogue obtenu par un procédé de fabrication tel que défini dans l'une quelconque des revendications 15 à 18, est lié et fixé sur la surface du support solide.

20. Procédé de liaison et fixation d'un oligonucléotide ou polynucléotide complémentaire, dans lequel le support solide ayant la partie convexe et concave à laquelle le dérivé nucléotidique ou son analogue est lié tel que défini dans la revendication 19, est mis en contact avec un oligonucléotide ou un polynucléotide présentant une complémentarité au dit dérivé nucléotidique ou son analogue fixé en présence d'un milieu aqueux.

21. Procédé tel que défini dans la revendication 20, dans lequel un marqueur détectable est lié au dit oligonucléotide ou polynucléotide complémentaire.

22. Support solide auquel et sur lequel un oligonucléotide ou un polynucléotide présentant une complémentarité est lié et fixé, dans lequel le support solide ayant la partie convexe et concave à laquelle le dérivé nucléotidique ou son analogue est lié tel que défini dans la revendication 19, un oligonucléotide ou un polynucléotide présentant une complémentarité au dit dérivé nucléotidique ou son analogue est lié suivant un mécanisme de complémentarité.

23. Support solide tel que défini dans la revendication 22, dans lequel un marqueur détectable est lié au dit oligonucléotide ou polynucléotide présentant la complémentarité.

24. Procédé d'identification ou de criblage d'un gène, dans lequel le support solide ayant une partie convexe et concave sur la surface de laquelle le dérivé nucléotidique ou son analogue est lié et fixé tel que défini dans la revendication 18, ou le support solide auquel et sur lequel l'oligonucléotide ou le polynucléotide complémentaire est lié et fixé tel que défini dans la revendication 20, est utilisé.

25. Puce pour matériel biologique, dans laquelle A, qui représente un résidu d'au moins une protéine ou substance de liaison à une protéine, est lié à un support solide ayant une partie convexe et concave par liaison covalente par l'intermédiaire d'un groupe sulfonyle comme la formule suivante (I) :
support solide-L-SO₂-X-A (I)
dans la formule (I), L représente un groupe de liaison ; X représente -CR¹(R²)-R³(R⁴)-; chacun parmi R¹, R², R³ et R⁴ représente indépendamment l'un de l'autre un atome d'hydrogène, un groupe alkyle ayant 1 à 6 atomes de carbone, un groupe aryle ayant 6 à 20 atomes de carbone ou un groupe aralkyle ayant 7 à 26 atomes de carbone au total contenant une chaîne alkyle ayant 1 à 6 atomes de carbone ; et A représente un résidu d'une protéine ou substance de liaison à une protéine à l'exception de l'acide nucléique, dans laquelle les parties convexes et concaves sont générées par revêtement du support solide avec une substance particulaire.

26. Puce telle que définie dans la revendication 25, dans laquelle ladite protéine ou substance de liaison à une protéine liée à la surface est un anticorps, un fragment d'anticorps, un ligand, un antigène, un haptène ou un récepteur.

27. Puce telle que définie dans la revendication 25, dans laquelle ladite protéine ou substance de liaison à une protéine liée à la surface est représentée par les avidines.

28. Puce telle que définie dans la revendication 27, dans laquelle les avidines sont une avidine, une streptoavidine ou des corps altérés de celles-ci qui sont capables de former un complexe stable avec une biotine.

29. Puce telle que définie dans la revendication 25, dans laquelle ladite protéine liée à la surface est une protéine de reconnaissance de l'acide nucléique.

30. Puce telle que définie dans la revendication 29, dans laquelle ladite protéine de reconnaissance de l'acide nucléique est une protéine de reconnaissance de l'ADN double brin.

31. Puce telle que définie dans la revendication 30, dans laquelle ladite protéine de reconnaissance de l'ADN double brin est un anticorps de reconnaissance de l'ADN double brin.

32. Puce telle que définie dans la revendication 30, dans laquelle ladite protéine de reconnaissance de l'ADN double brin est un facteur de transcription de l'ADN.

33. Puce telle que définie dans la revendication 30, dans laquelle ladite protéine de reconnaissance de l'ADN double brin est une protéine ayant un motif de doigt de zinc ou un motif de doigt de cycle.

34. Puce telle que définie dans l'une quelconque des revendications 25 à 33, dans laquelle ladite partie convexe et concave comprend une particule formée par une substance inorganique.

35. Puce telle que définie dans la revendication 34, dans laquelle ladite partie convexe et concave comprend une particule contenant du silicium, de l'alumine ou du titane dont le diamètre de particule moyen est de 50 µm ou moins.

36. Puce telle que définie dans l'une quelconque des revendications 25 à 33, dans laquelle ladite partie convexe et concave est formée par une substance organique.

37. Puce telle que définie dans la revendication 36, dans laquelle ladite substance organique comprend un polymère de haut poids moléculaire ou un agrégat d'un polymère de haut poids moléculaire.

38. Puce telle que définie dans la revendication 25, dans laquelle ledit support solide est une surface en verre, en matières plastiques, d'électrode ou une surface de puce de détecteur.

39. Procédé de détection d'une substance cible, comprenant les étapes consistant à :
mettre en contact la puce définie dans la revendication 25 avec un échantillon contenant une substance cible qui se lie spécifiquement à ladite protéine ou substance de liaison à une protéine à l'exception de l'acide nucléique supporté sur la surface de ladite puce ; et
détecter la formation de liaison réciproque entre ladite protéine ou substance de liaison à une protéine et ladite substance cible.

40. Procédé de détection d'une substance cible tel que défini dans la revendication 39, dans lequel ladite substance cible est marquée avec au moins un composant capable de générer un signal détectable.

41. Procédé de détection d'une substance cible tel que défini dans la revendication 39, comprenant une étape consistant à mettre en oeuvre un processus de blocage de la puce avec une solution aqueuse d'un acide aminé, d'un peptide ou d'une protéine.

42. Procédé de fabrication d'une puce telle que définie dans la revendication 25, comprenant une étape dans laquelle un support solide ayant une partie convexe et concave contenant un groupement vinyl-sulfonyle ou son groupe précurseur réactif représenté par la formule (II) suivante sur sa surface, est mis en contact avec au moins une protéine ou substance de liaison à une protéine ayant un groupe réactif qui forme une liaison covalente par réaction avec ledit groupement vinyl-sulfonyle ou son groupe précurseur réactif :
-L-SO₂-X' (II)
dans la formule (II) décrite ci-dessus, L représente un groupe de liaison qui se lie à -L-SO₂-X' sur un support solide ; X' représente -CR¹=CR²(R³) ou -CH(R¹)-CR²(R³) (Y) ; chacun parmi R¹, R² et R³ représente indépendamment l'un de l'autre un atome d'hydrogène, un groupe alkyle ayant 1 à 6 atomes de carbone, un groupe aryle ayant 6 à 20 atomes de carbone ou un groupe aralkyle ayant 7 à 26 atomes de carbone au total contenant une chaîne alkyle ayant 1 à 6 atomes de carbone ; et Y représente un groupe qui est substitué par un réactif nucléophile ou un groupe qui est éliminé comme un « HY » par une base.

43. Procédé de fabrication d'une puce tel que défini dans la revendication 42, dans lequel ladite protéine ou substance de liaison à une protéine liée à la surface est un anticorps, un fragment d'anticorps, un ligand, un antigène, un haptène ou un récepteur.

44. Procédé de fabrication d'une puce tel que défini dans la revendication 42, dans lequel ladite protéine or substance de liaison à une protéine liée à la surface est représentée par les avidines.

45. Procédé de fabrication d'une puce tel que défini dans la revendication 44, dans lequel les avidines sont une avidine, une streptoavidine ou des corps altérés de celles-ci qui sont capables de former un complexe stable avec une biotine.

46. Procédé de fabrication d'une puce tel que défini dans la revendication 42, dans lequel ladite protéine liée à la surface est une protéine de reconnaissance de l'acide nucléique.

47. Procédé de fabrication d'une puce tel que défini dans la revendication 46, dans lequel ladite protéine de reconnaissance de l'acide nucléique est une protéine de reconnaissance de l'ADN double brin.

48. Procédé de fabrication d'une puce tel que défini dans la revendication 47, dans lequel ladite protéine de reconnaissance de l'ADN double brin est un anticorps de reconnaissance de l'ADN double brin.

49. Procédé de fabrication d'une puce tel que défini dans la revendication 47, dans lequel ladite protéine de reconnaissance de l'ADN double brin est un facteur de transcription de l'ADN.

50. Procédé de fabrication d'une puce tel que défini dans la revendication 47, dans lequel ladite protéine de reconnaissance de l'ADN double brin est une protéine ayant un motif de doigt de zinc ou un motif de doigt de cycle.

51. Procédé de fabrication d'une puce tel que défini dans la revendication 42, dans lequel ladite partie convexe et concave comprend une particule formée par une substance inorganique.

52. Procédé de fabrication d'une puce telle que définie dans la revendication 34, dans lequel ladite partie convexe et concave comprend une particule contenant du silicium, de l'alumine ou du titane dont le diamètre de particule moyen est de 50 µm ou moins.

53. Procédé de fabrication d'une puce tel que défini dans la revendication 42, dans lequel ladite partie convexe et concave est formée par une substance organique.

54. Procédé de fabrication d'une puce tel que défini dans la revendication 53, dans lequel ladite substance organique est un polymère de haut poids moléculaire ou un agrégat d'un polymère de haut poids moléculaire.

55. Procédé de fabrication d'une puce tel que défini dans la revendication 42, dans lequel un support solide est une surface en verre, en matières plastiques, d'électrode ou une surface de puce de détecteur.

56. Procédé de fabrication d'une puce tel que défini dans la revendication 42, comprenant les étapes consistant à :
fixer au moins une protéine ou substance de liaison à une protéine sur un support solide ayant des parties convexes et concaves par mise en contact de ladite protéine ou substance de liaison à une protéine sur ledit support solide ; et
mettre en oeuvre le processus de blocage d'un groupement vinyl-sulfonyle libre ou son groupe précurseur réactif localisé sur la surface dudit support solide avec une solution aqueuse d'un acide aminé, d'un peptide ou d'une protéine.
